# EUROPÄISCHE PATENTSCHRIFT

(11) **EP 4 233 726 B1**
(45) Veröffentlichungstag und Bekanntmachung des Hinweises auf die Patenterteilung: **14.10.2026**
(21) Anmeldenummer: 22158528.4
(22) Anmeldetag: 24.02.2022
(51) Int. Cl.: A61B 6/00, A61B 8/00, G16H 30/00, G16H 30/40, G06N 3/02

(54) **VORHERSAGE EINER REPRÄSENTATION EINES UNTERSUCHUNGSBEREICHS EINES UNTERSUCHUNGSOBJEKTS NACH DER APPLIKATION UNTERSCHIEDLICHER MENGEN EINES KONTRASTMITTELS**
PREDICTION OF A REPRESENTATION OF AN AREA OF AN OBJECT TO BE EXAMINED AFTER THE APPLICATION OF DIFFERENT AMOUNTS OF A CONTRAST AGENT
PRÉDICTION D'UNE REPRÉSENTATION D'UNE ZONE D'EXAMEN D'UN OBJET D'EXAMEN APRÈS L'APPLICATION DE DIFFÉRENTES QUANTITÉS D'UN AGENT DE CONTRASTE

(43) Veröffentlichungstag der Anmeldung: 30.08.2023
(73) Patentinhaber: Bayer Aktiengesellschaft, 51373 Leverkusen (DE)
(72) Erfinder: LENGA, Matthias, 24114 Kiel (DE); SCHÜTZ, Gunnar, 13467 Berlin (DE); VÖLKENING, Stephan, 12161 Berlin (DE)
(74) Vertreter: BIP Patents

(56) Entgegenhaltungen:
- EP-A1- 3 739 522
- WO-A1-2021/044153
- US-A1- 2020 294 288

## Beschreibung

Die vorliegende Erfindung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage von Repräsentationen eines Untersuchungsbereich nach der Applikation unterschiedlicher Mengen eines Kontrastmittels.

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In einem ersten Schritt wird ein Trainingsdatensatz erzeugt. Der Trainingsdatensatz umfasst für jede Person einer Vielzahl an Personen i) eine native radiologische Aufnahme (*zero-contrast image*)*,* ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*)*.*

In einem zweiten Schritt wird ein künstliches neuronales Netz trainiert, für jede Person des Trainingsdatensatzes auf Basis der nativen Aufnahme und der Aufnahme nach Applikation einer geringen Menge an Kontrastmittel eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*)*.*

In einem dritten Schritt kann das trainierte künstliche neuronale Netz verwendet werden, für eine neue Person auf Basis einer nativen Aufnahme und einer radiologischen Aufnahme nach der Applikation einer geringen Menge an Kontrastmittel eine künstliche radiologische Aufnahme vorherzusagen, die den aufgenommenen Bereich so zeigt wie er aussehen würde, wenn eine Standardmenge an Kontrastmittel appliziert worden wäre.

Bei dem in WO2019/074938A1 offenbarten Verfahren wird ein künstliches neuronales Netzwerk trainiert, die native radiologische Aufnahme und die radiologische Aufnahme nach der Applikation einer geringen Menge an Kontrastmittel auf die radiologische Aufnahme nach der Applikation einer Standardmenge an Kontrastmittel abzubilden (engl. *mapping*)*.* Das künstliche neuronale Netz wird aber nicht trainiert, einen zunehmenden Einfluss von Kontrastmittel auf eine radiologische Aufnahme zu lernen und kann damit nicht eingesetzt werden, um radiologische Aufnahmen zu erzeugen, die einen Untersuchungsbereichs nach der Applikation unterschiedlicher Mengen an Kontrastmittel zeigen.

Insbesondere ist das in WO2019/074938A1 beschriebene künstliche neuronale Netzwerk nicht geeignet, radiologische Aufnahmen zu erzeugen, die einen Untersuchungsbereich nach einer Applikation einer größeren Menge als der Standardmenge an Kontrastmittel zu zeigen.

Ferner sind im Fall des in WO2019/074938A1 beschriebenen Verfahrens zur Erzeugung einer radiologischen Aufnahme, die einen Untersuchungsbereich nach einer Applikation der Standardmenge eines Kontrastmittels zeigt, stets mindestens zwei radiologische Aufnahmen (eine native radiologische Aufnahme und eine radiologische Aufnahme nach der Applikation einer geringen Menge des Kontrastmittels) erforderlich.

WO2021044153A1 offenbart ein Verfahren und eine Vorrichtung zur Verbesserung von Magnetresonanzbildern, um kontrastverstärkte Bilder zu erzeugen, ohne dass einem Patienten ein Kontrastmittel verabreicht werden muss. Die in Bezug auf WO2019/074938A1 genannten Nachteile treffen auch auf den in WO2021044153A1 offenbarten Ansatz zu.

US2020/294288A1 offenbart ein Verfahren und ein System zur Rekonstruktion eines kontrastverstärkten CT-Ausgangsbildes aus einem nicht verstärkten CT-Eingangsbild. Die in Bezug auf WO2019/074938A1 genannten Nachteile treffen auch auf den in US2020/294288A1 offenbarten Ansatz zu.

EP3739522A1 offenbart ein System zur Kontrastverstärkung in radiologischen Aufnahmen. Die in Bezug auf WO2019/074938A1 genannten Nachteile treffen auch auf den in EP3739522A1 offenbarten Ansatz zu.

Ausgehend vom beschriebenen Stand der Technik stellte sich die Aufgabe, ein Modell des maschinellen Lernens bereitzustellen, mit dem künstliche radiologische Aufnahmen erzeugt werden können, die einen Untersuchungsbereich nach der Applikation unterschiedlicher Mengen eines Kontrastmittels zeigen. Das Modell soll ferner prinzipiell in der Lage sein, künstliche radiologische Aufnahmen nach der Applikation unterschiedlicher Mengen an Kontrastmittel auf Basis einer einzigen messtechnisch erzeugten Aufnahme zu erzeugen.

Diese Aufgaben werden durch die Gegenstände der unabhängigen Patentansprüche gelöst. Bevorzugte Ausführungsformen finden sich in den abhängigen Patentansprüchen, der vorliegenden Beschreibung sowie in den Zeichnungen.

Ein Gegenstand der vorliegenden Erfindung ist ein Verfahren umfassend die Schritte:
- Empfangen einer Repräsentation R*ₚ* eines Untersuchungsbereichs,
   ∘ wobei die Repräsentation R*ₚ* den Untersuchungsbereich ohne Kontrastmittel oder nach Applikation einer Menge *aₚ* eines Kontrastmittels repräsentiert, wobei *p* eine ganze Zahl ist, die kleiner als *n* ist, wobei *n* eine ganze Zahl größer als 2 ist,
   ∘ wobei der Untersuchungsbereich die Leber oder ein Teil der Leber eines Menschen ist, wobei die Repräsentation R*ₚ* eine radiologische Aufnahme ist,
- Zuführen der Repräsentation R*ₚ* einem trainierten Modell des maschinellen Lernens,
   ∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- Empfangen, von dem Modell des maschinellen Lernens, einer oder mehrerer vorhergesagter Repräsentationen *R_{p+q}*^{*} des Untersuchungsbereichs, wobei jede der einen oder der mehreren vorhergesagten Repräsentationen *R_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
- Ausgeben und/oder Speichern und/oder Übermitteln der einen oder der mehreren vorhergesagten Repräsentationen R*_{p+q}*^{*}.

Ein weiterer Gegenstand der vorliegenden Erfindung ist ein Computersystem umfassend
- eine Eingabeeinheit,
- eine Steuer- und Recheneinheit und
- eine Ausgabeeinheit,
wobei die Steuer- und Recheneinheit konfiguriert ist,
- eine Repräsentation R*ₚ* eines Untersuchungsbereichs zu empfangen,
   ∘ wobei die Repräsentation R*ₚ* den Untersuchungsbereich ohne Kontrastmittel oder nach einer Applikation einer Menge *aₚ* eines Kontrastmittels repräsentiert, wobei *p* eine ganze Zahl ist, die kleiner als *n* ist, wobei *n* eine ganze Zahl größer als 2 ist,
   ∘ wobei der Untersuchungsbereich die Leber oder ein Teil der Leber eines Menschen ist, wobei die Repräsentation R*ₚ* eine radiologische Aufnahme ist,
- die Repräsentation R*ₚ* einem trainierten Modell des maschinellen Lernens zuzuführen,
   ∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- von dem Modell des maschinellen Lernens eine oder mehrere vorhergesagte Repräsentationen R*_{p+q}*^{*} des Untersuchungsbereichs zu empfangen, wobei jede der einen oder der mehreren vorhergesagten Repräsentationen R*_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
- die eine oder die mehreren vorhergesagten Repräsentationen R*_{p+q}*^{*} zu speichern und/oder auszugeben und/oder an ein separates Computersystem zu übermitteln.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen einer Repräsentation R*ₚ* eines Untersuchungsbereichs,
   ∘ wobei die Repräsentation R*ₚ* den Untersuchungsbereich ohne Kontrastmittel oder nach Applikation einer Menge *aₚ* eines Kontrastmittels repräsentiert, wobei *p* eine ganze Zahl ist, die kleiner als *n* ist, wobei *n* eine ganze Zahl größer als 2 ist,
   ∘ wobei der Untersuchungsbereich die Leber oder ein Teil der Leber eines Menschen ist, wobei die Repräsentation R*ₚ* eine radiologische Aufnahme ist,
- Zuführen der Repräsentation R*ₚ* einem trainierten Modell des maschinellen Lernens,
   ∘ wobei das Modell des maschinellen Lernens auf Basis von Trainingsdaten trainiert worden ist, ausgehend von einer ersten Referenz-Repräsentation R₁ eine Zahl *n-*1 von vorhergesagten Referenz-Repräsentationen R₂^{*} bis R*ₙ*^{*} zu erzeugen,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
   • Empfangen, von dem Modell des maschinellen Lernens, einer oder mehrerer vorhergesagter Repräsentationen *R_{p+q}*^{*} des Untersuchungsbereichs, wobei jede der einen oder der mehreren vorhergesagten Repräsentationen *R_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
   • Ausgeben und/oder Speichern und/oder Übermitteln der einen oder der mehreren vorhergesagten Repräsentationen R*_{p+q}*^{*}.

Ein weiterer Gegenstand der vorliegenden Erfindung ist eine Verwendung eines Kontrastmittels in einem radiologischen Untersuchungsverfahren, wobei das radiologische Untersuchungsverfahren die folgenden Schritte umfasst:
- Empfangen einer Repräsentation R*ₚ* eines Untersuchungsbereichs,
   ∘ wobei die Repräsentation R*ₚ* den Untersuchungsbereich ohne Kontrastmittel oder nach Applikation einer Menge *aₚ* des Kontrastmittels repräsentiert, wobei *p* eine ganze Zahl ist, die kleiner als *n* ist, wobei *n* eine ganze Zahl größer als 2 ist,
   ∘ wobei der Untersuchungsbereich die Leber oder ein Teil der Leber eines Menschen ist, wobei die Repräsentation R*ₚ* eine radiologische Aufnahme ist,
- Zuführen der Repräsentation R*ₚ* einem trainierten Modell des maschinellen Lernens,
   ∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei *k* ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- Empfangen, von dem Modell des maschinellen Lernens, einer oder mehrerer vorhergesagter Repräsentationen R*_{p+q}*^{*} des Untersuchungsbereichs, wobei jede der einen oder der mehreren vorhergesagten Repräsentationen R*_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
- Ausgeben und/oder Speichern und/oder Übermitteln der einen oder der mehreren vorhergesagten Repräsentationen R*_{p+q}*^{*}.

Ein weiterer Gegenstand der vorliegenden Erfindung ist ein Kit umfassend ein Kontrastmittel und ein Computerprogrammprodukt, wobei das Computerprogrammprodukt ein Computerprogramm umfasst, das in einen Arbeitsspeicher eines Computersystems geladen werden kann und dort das Computersystem dazu veranlasst, folgende Schritte ausführen:
- Empfangen einer Repräsentation R*ₚ* eines Untersuchungsbereichs,
   ∘ wobei die Repräsentation R*ₚ* den Untersuchungsbereich ohne Kontrastmittel oder nach Applikation einer Menge *aₚ* des Kontrastmittels repräsentiert, wobei *p* eine ganze Zahl ist, die kleiner als *n* ist, wobei *n* eine ganze Zahl größer als 2 ist,
   ∘ wobei der Untersuchungsbereich die Leber oder ein Teil der Leber eines Menschen ist, wobei die Repräsentation R*ₚ* eine radiologische Aufnahme ist,
- Zuführen der Repräsentation R*ₚ* einem trainierten Modell des maschinellen Lernens,
   ∘ wobei das Modell des maschinellen Lernens auf Basis von Trainingsdaten trainiert worden ist, ausgehend von einer ersten Referenz-Repräsentation R₁ eine Zahl *n-*1 von vorhergesagten Referenz-Repräsentationen R₂^{*} bis R*ₙ*^{*} zu erzeugen,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- Empfangen, von dem Modell des maschinellen Lernens, einer oder mehrerer vorhergesagter Repräsentationen R*_{p+q}*^{*} des Untersuchungsbereichs, wobei jede der einen oder der mehreren vorhergesagten Repräsentationen R*_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
- Ausgeben und/oder Speichern und/oder Übermitteln der einen oder der mehreren vorhergesagten Repräsentationen R*_{p+q}*^{*}.

Die Erfindung wird nachstehend näher erläutert, ohne zwischen den Erfindungsgegenständen (Verfahren, Computersystem, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt, Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Mit Hilfe der vorliegenden Erfindung können Repräsentationen eines Untersuchungsbereichs eines Untersuchungsobjekts vorhergesagt werden.

Das "Untersuchungsobjekt" ist ein Lebewesen, ein Mensch.

Der "Untersuchungsbereich" ist ein Teil des Untersuchungsobjekts.

Der Untersuchungsbereich ist die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Eine "Repräsentation des Untersuchungsbereichs" ist das Ergebnis einer radiologischen Untersuchung.

Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt.

Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine computertomographische Untersuchung oder eine magnetresonanztomographische Untersuchung.

Computertomographie (CT) ist ein Röntgenverfahren, mit dem der menschliche Körper in Querschnittbildern (Schnittbildverfahren) dargestellt wird. Im Vergleich zu einer herkömmlichen Röntgenaufnahme, auf der üblicherweise nur grobe Strukturen und Knochen erkennbar sind, wird in CT-Aufnahmen auch Weichteilgewebe mit geringen Kontrastunterschieden detailliert erfasst. Eine Röntgenröhre erzeugt einen sogenannten Röntgenfächerstrahl, der den Körper durchdringt und innerhalb des Körpers durch die verschiedenen Strukturen, wie Organe und Knochen, unterschiedlich stark abgeschwächt wird. Die Empfangsdetektoren gegenüber dem Röntgenstrahler empfangen die unterschiedlich starken Signale und leiten sie an einen Computer weiter, der aus den empfangenen Daten Schichtbilder des Körpers zusammensetzt. Computertomografische Aufnahmen (CT-Aufnahmen) können in 2D oder auch in 3D betrachtet werden. Zur besseren Abgrenzbarkeit von Strukturen innerhalb des Körpers des Menschen (z.B. Gefäße), kann vor der Erzeugung von CT-Aufnahmen ein Kontrastmittel z.B. in eine Vene gespritzt werden.

Die Magnetresonanztomographie, abgekürzt MRT oder MRI (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MR-Daten liegen zunächst als Rohdaten im Frequenzraum vor, und können durch anschließende inverse Fourier-Transformation in den Ortsraum (Bildraum) transformiert werden.

Auch in der Magnetresonanztomographie können Kontrastmittel eingesetzt werden, um eine Kontrastverstärkung zu erreichen.

"Kontrastmittel" sind Substanzen oder Gemische von Substanzen, die die Darstellung von Strukturen und Funktionen des Körpers bei bildgebenden radiologischen Verfahren verbessern.

Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

Eine Repräsentation des Untersuchungsbereichs im Sinne der vorliegenden Erfindung kann ein Computertomogramm, eine MRT-Aufnahme, ein Ultraschallbild oder dergleichen sein.

Eine Repräsentation des Untersuchungsbereichs im Sinne der vorliegenden Erfindung kann eine Repräsentation im Ortsraum (Bildraum) oder eine Repräsentation im Frequenzraum oder eine andere Repräsentation sein. Vorzugsweise liegen Repräsentationen des Untersuchungsbereichs in einer Ortsraum-Darstellung oder in einer Form vor, die sich in eine Ortsraum-Darstellung umwandeln (transformieren) lässt.

Eine Repräsentation im Sinne der vorliegenden Erfindung zeigt den Untersuchungsbereich vor oder nach der Applikation einer Menge eines Kontrastmittels.

Unter dem Begriff "Menge" kann die absolute Menge, die einem Untersuchungsobjekt verabreicht wurde, verstanden werden (z.B. gemessen in Kilogramm oder Mol oder Liter); es kann sich bei der "Menge" aber auch um eine verabreichte Dosis handeln, wie beispielsweise eine Menge an Kontrastmittel (z.B. gemessen in Kilogramm oder Mol oder Liter) pro Kilogramm Körpergewicht des Untersuchungsobjekts. Es kann sich bei dem Begriff "Menge" aber auch um eine Konzentration handeln, die sich zumindest für einen definierten Zeitpunkt nach der Applikation des Kontrastmittels in dem Untersuchungsobjekt einstellt. Weitere Mengen-Definitionen sind denkbar.

Vorzugsweise handelt es sich bei dem Kontrastmittel um ein MR-Kontrastmittel. MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*)*.* Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Vorzugsweise handelt es sich bei dem MR-Kontrastmittel um ein hepatobiliäres Kontrastmittel. Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist unter dem Markennamen Primovist^{®} und Eovist^{®} kommerziell erhältlich ist.

Das Kontrastmittel kann gewichtsadaptiert in Form einer Bolusinjektion beispielsweise in eine Armvene appliziert werden.

Mit Hilfe der vorliegenden Erfindung können Repräsentationen eines Untersuchungsbereichs nach der Applikation unterschiedlicher Mengen eines Kontrastmittels vorhergesagt werden. Dabei kann die Vorhersage prinzipiell auf Basis einer einzigen Repräsentation erfolgen, die den Untersuchungsbereich ohne Kontrastmittel oder nach der Applikation einer ersten Menge eines Kontrastmittels repräsentiert.

Die Vorhersage erfolgt mit Hilfe eines Modells des maschinellen Lernens.

Ein "Modell des maschinellen Lernens" kann als eine computerimplementierte Datenverarbeitungsarchitektur verstanden werden. Das Modell kann Eingangsdaten empfangen und Ausgangsdaten auf der Grundlage dieser Eingangsdaten und Modell-Parametern liefern. Das Modell kann durch Training eine Beziehung zwischen den Eingabedaten und den Ausgabedaten erlernen. Beim Training können Modell-Parameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modell-Parameter werden so verändert, dass die Abweichungen zwischen den Ausgabedaten und den Zieldaten auf ein (definiertes) Minimum reduziert werden.

Die Abweichungen können mit Hilfe einer Fehlerfunktion (engl.: *loss function*) quantifiziert werden. Eine solche Fehlerfunktion kann verwendet werden, um einen Fehlerwert (engl.: *loss value*) für ein gegebenes Paar von Ausgabedaten und Zieldaten zu berechnen. Das Ziel des Trainingsprozesses kann darin bestehen, die Parameter des maschinellen Lernmodells so zu verändern (anzupassen), dass der Fehlerwert für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehlerwert bedeuten, dass ein oder mehrere Modell-Parameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, ausgehend von einer ersten Repräsentation, die einen Untersuchungsbereich ohne Kontrastmittel oder nach der Applikation einer ersten Menge eines Kontrastmittels repräsentiert, eine Folge von Repräsentationen vorherzusagen, die den Untersuchungsbereich nach der Applikation unterschiedlicher Mengen des Kontrastmittels repräsentieren.

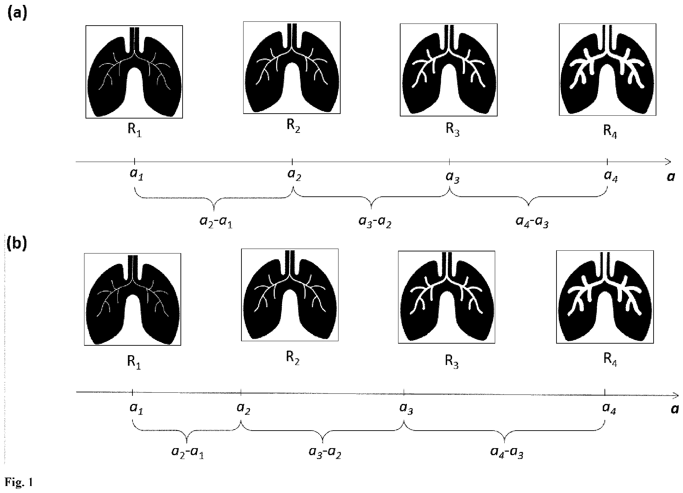

Die unterschiedlichen Mengen an Kontrastmittel bilden eine Folge, in der die Kontrastmittelmengen beispielsweise zunehmen oder abnehmen können. Dies sei anhand von Fig. 1 erläutert.

Fig. 1 zeigt beispielhaft und schematisch vier Repräsentationen eines Untersuchungsbereichs nach der Applikation unterschiedlicher Mengen eines Kontrastmittels. Bei dem Untersuchungsbereich handelt es sich um die Lunge eines Menschen. Die vier Repräsentationen R₁, R₂, R₃ und R₄ sind entlang einer Achse aufgereiht, die die Menge *a* an Konzentrationsmittel, die dem Untersuchungsobjekt jeweils verabreicht worden ist, angibt. Die Repräsentation R₁ zeigt also den Untersuchungsbereich nach einer Applikation einer ersten Menge *a*₁ des Konzentrationsmittels; die Repräsentation R₂ zeigt den Untersuchungsbereich nach einer Applikation einer zweiten Menge *a₂* des Konzentrationsmittels; R₃ zeigt den Untersuchungsbereich nach einer Applikation einer dritten Menge *a*₃ des Konzentrationsmittels; R₄ zeigt den Untersuchungsbereich nach einer Applikation einer vierten Menge *a*₄ des Konzentrationsmittels. Die erste Menge *a*₁ kann größer oder gleich Null sein, die zweite Menge *a*₂, die dritte Menge *a*₃ und die vierte Menge *a*₄ sind in dem vorliegenden Beispiel jeweils größer als Null. Im vorliegenden Beispiel ist die zweite Menge *a*₂ größer als die erste Menge *a*₁, die dritte Menge *a*₃ größer als die zweite Menge *a*₂ und die vierte Menge *a*₄ größer als die dritte Menge *a*₃: 0 ≤ *a*₁ *< a*₂ *< a*₃ *< a*₄

In dem in Fig. 1 gezeigten Beispiel bilden die Kontrastmittelmengen also eine Folge zunehmender Kontrastmittelmengen.

Dies zeigt sich in Fig. 1 in den Repräsentationen R₁ bis R₄ durch eine zunehmende Kontrastverstärkung der Blutgefäße gegenüber dem umliegenden Gewebe.

In dem in Fig. 1 (a) gezeigten Beispiel nimmt die Menge um einen gleichbleibenden Betrag zu, d.h. die Differenz der Mengen zweier unmittelbar aufeinanderfolgender Mengen ist für alle unmittelbar aufeinanderfolgenden Mengen gleich: *a*₂ *- a*₁ *= a*₃ *- a*₂ *= a*₄ *- a*₃
In einer Folge von Kontrastmittelmengen können die Mengen jedoch auch um einen unterschiedlichen Betrag zunehmen. In dem in Fig. 1 (b) gezeigten Beispiel nimmt die Menge um einen zunehmenden Betrag zu: *a*₂ *- a*₁ *< a*₃ *- a*₂ *< a*₄ *- a*₃
Es ist aber auch möglich, dass die Mengen um einen abnehmenden Betrag zunehmen: *a*₂ *- a*₁ *> a*₃ *- a*₂ *> a*₄ *- a*₃
Es ist aber auch möglich, dass die Repräsentationen anders entlang der Mengen-Achse verteilt sind.

Ferner ist es möglich, dass die Kontrastmittelmengen eine Folge abnehmender Mengen bildet oder eine andere Folge bildet.

Das Modell des maschinellen Lernens kann anhand von Trainingsdaten trainiert werden, ausgehend von der ersten Repräsentation R₁ nacheinander die Repräsentationen R₂, R₃ und R₄ vorherzusagen. Mit anderen Worten: das Modell des maschinellen Lernens kann die Folge von Kontrastmittelmengen lernen, um sie dann auf neue Repräsentationen anzuwenden.

Das Training wird nachfolgend der Einfachheit halber zunächst auf Basis von drei Repräsentationen erläutert. Anschließend erfolgt eine Erweiterung auf eine beliebige Zahl *n* an Repräsentationen, wobei *n* eine ganze Zahl größer als zwei ist.

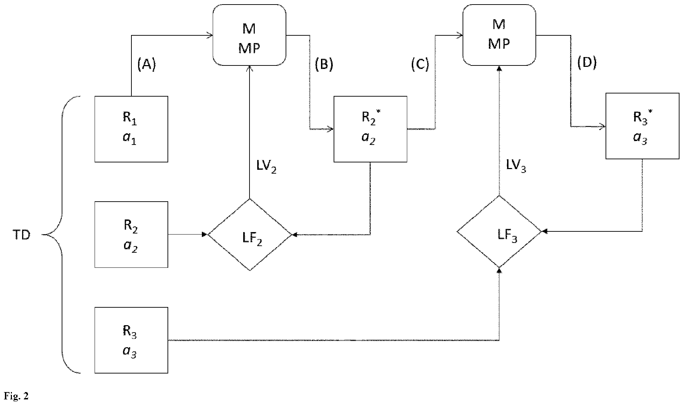

Bei der nachfolgenden Erläuterung des Trainings auf Basis von drei Repräsentationen wird auf Fig. 2 Bezug genommen.

Fig. 2 zeigt drei Repräsentationen, eine erste Repräsentation R₁, eine zweite Repräsentation R₂ und eine dritte Repräsentation R₃. Die erste Repräsentation R₁ repräsentiert einen Untersuchungsbereich eines Untersuchungsobjekts ohne Kontrastmittel oder nach einer Applikation einer ersten Menge *a*₁ eines Kontrastmittels, die zweite Repräsentation R₂ repräsentiert den Untersuchungsbereich nach einer Applikation einer zweiten Menge *a*₂ des Kontrastmittels und R₃ repräsentiert den Untersuchungsbereich nach einer Applikation einer dritten Menge *a*₃ des Kontrastmittels. Die Kontrastmittelmengen *a*₁, *a*₂ und *a*₃ können beispielsweise eine Folge zunehmenden Mengen an Kontrastmittel bilden (0 ≤ *a*₁ *< a*₂ *< a*₃).

Die drei Repräsentationen R₁, R₂ und R₃ bilden einen Datensatz innerhalb von Trainingsdaten TD. Die Trainingsdaten TD umfassen eine Vielzahl solcher Datensätze. Der Begriff Vielzahl bedeutet vorzugsweise mehr als 100. Jeder Datensatz umfasst üblicherweise drei Repräsentationen des Untersuchungsbereichs nach der Applikation der jeweiligen Mengen des Kontrastmittels (die im Fall von *a*₁ auch Null sein kann). Dabei ist der Untersuchungsbereich bei allen Repräsentationen der gleiche; lediglich das Untersuchungsobjekt kann variieren; üblicherweise stammt jeder Datensatz von einem anderen Untersuchungsobjekt. Die in diesem Absatz getätigten Aussagen gelten analog auch für andere Ausführungsformen und nicht nur in Bezug auf das in Fig. 2 gezeigte Beispiel.

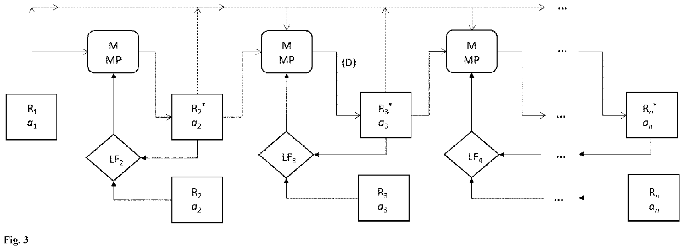

Die Repräsentationen, die zum Trainieren des Modells des maschinellen Lernens verwendet werden, werden in dieser Beschreibung auch als Referenz-Repräsentationen bezeichnet; zur besseren Verständlichkeit/Lesbarkeit wird in den Erläuterungen zu Fig. 2 und Fig. 3 aber auf den Zusatz "Referenz-" verzichtet.

Mittels des Modells des maschinellen Lernens erzeugte (vorhergesagte) Repräsentationen werden in dieser Beschreibung überwiegend durch ein hochgestelltes Stern-Symbol * gekennzeichnet.

In einem ersten Schritt (A) wird die erste Repräsentation R₁ einem Modell des maschinellen Lernens M zugeführt. Das Modell des maschinellen Lernens M ist konfiguriert, auf Basis der ersten Repräsentation R₁ und auf Basis von Modellparametern MP eine Ausgabe R₂^{*} zu erzeugen (Schritt (B)). Die Ausgabe R₂^{*} soll der zweiten Repräsentation R₂ möglichst nahekommen; im Idealfall kann die Ausgabe R₂^{*} nicht von der zweiten Repräsentation R₂ unterschieden werden. Mit anderen Worten: bei der Ausgabe R₂^{*} handelt es sich um eine vorhergesagte zweite Repräsentation. Die Ausgabe R₂^{*} wird mit der (realen) zweiten Repräsentation R₂ verglichen und die Abweichungen werden mit Hilfe einer Fehlerfunktion LF₂ quantifiziert. Für jedes Paar von Ausgabe R₂^{*} und zweiter Repräsentation R₂ kann ein Fehlerwert LV₂ mittels der Fehlerfunktion LF₂ berechnet werden.

Beispiele für Fehlerfunktionen, die allgemein (nicht beschränkt auf das Beispiel in Fig. 2) zur Ausführung der vorliegenden Erfindung verwendet werden können, sind L1-Fehlerfunktion (*L1 loss*)*,* L2-Fehlerfunktion (*L2 loss*)*,* Lp-Fehlerfunktion (Lp loss), Strukturähnlichkeitsindexmaß (*structural similarity index measure* (SSIM)), VGG-Fehlerfunktion (VGG loss), Wahrnehmungs-Fehlerfunktion (perceptual loss) oder eine Kombination der oben genannten Funktionen. Weitere Einzelheiten über Fehlerfunktionen sind z.B. in der wissenschaftlichen Literatur zu finden (siehe z. B.: R. Mechrez et al.: The Contextual Loss for Image Transformation with Non-Aligned Data, 2018, arXiv:1803.02077v4; H. Zhao et al.: Loss Functions for Image Restoration with Neural Networks, 2018, arXiv:1511.08861v3).

Die Ausgabe R₂^{*} wird in Schritt (C) erneut dem Modell des maschinellen Lernens M zugeführt. Auch wenn Fig. 2 einen anderen Eindruck erwecken mag: bei dem Modell des maschinellen Lernens M, dem in Schritt (A) die erste Repräsentation R₁ und in Schritt (C) die vorhergesagte zweite Repräsentation R₂^{*} zugeführt werden, handelt es sich um dasselbe Modell. Das heißt, das Modell des maschinellen Lernens ist nicht nur konfiguriert, auf Basis der ersten Repräsentation eine vorhergesagte zweite Repräsentation zu erzeugen, sondern es ist auch konfiguriert, aus einer (vorhergesagten und/oder realen) zweiten Repräsentation eine Ausgabe R₃^{*} zu erzeugen, die der dritten Repräsentation R₃ möglichst nahekommt. Die Ausgabe R₃^{*} ist eine vorhergesagte dritte Repräsentation. Die Ausgabe R₃^{*} wird mit der dritten Repräsentation R₃ verglichen. Mit Hilfe einer Fehlerfunktion LF₃ kann die Abweichung zwischen der Ausgabe R₃^{*} und der dritten Repräsentation R₃ quantifiziert werden; es kann für jedes Paar einer dritten Repräsentation und einer vorhergesagten dritten Repräsentation ein Fehlerwert LV₃ ermittelt werden.

Vorzugsweise wird das Modell des maschinellen Lernens Ende-zu-Ende (engl. *end-to-end*) trainiert. Das bedeutet, dass das Modell des maschinellen Lernens gleichzeitig trainiert wird, eine vorhergesagte zweite Repräsentation auf Basis der ersten Repräsentation und eine vorhergesagte dritte Repräsentation auf Basis der vorhergesagten zweiten Repräsentation zu erzeugen. Vorzugsweise wird dazu eine Fehlerfunktion verwendet, die sowohl die Abweichungen zwischen der vorhergesagten zweiten Repräsentation und der zweiten Repräsentation als auch die Abweichungen zwischen der vorhergesagten dritten Repräsentation und der dritten Repräsentation berücksichtigt.

Es ist möglich, die Abweichungen zwischen der zweiten Repräsentation und der vorhergesagten zweiten Repräsentation mit Hilfe der Fehlerfunktion LF₂ zu quantifizieren und die Abweichungen zwischen der dritten Repräsentation und der vorhergesagten dritten Repräsentation mit Hilfe der Fehlerfunktion LF₃ zu quantifizieren. Eine Fehlerfunktion LF, die beide Abweichungen berücksichtigt, kann beispielsweise die Summe der einzelnen Fehlerfunktionen sein: LF = LF₂ + LF₃. Es ist auch möglich die Anteile, die die einzelnen Fehlerfunktionen LF₂ und LF₃ an der Gesamtfehlerfunktion LF haben, unterschiedlich zu gewichten: LF = w₂ · LF₂ + w₃ · LF₃, wobei w₂ und w₃ Gewichtsfaktoren sind, die beispielsweise Werte zwischen 0 und 1 annehmen können. Dabei kann der Wert Null für einen Gewichtsfaktor beispielsweise dann verwendet werden, wenn in einem Datensatz eine Repräsentation fehlt (mehr dazu ist in der Beschreibung weiter hinten zu finden).

Bei der (späteren) Verwendung des trainierten Modells des maschinellen Lernens zur Vorhersage kann es darum gehen, eine dritte Repräsentation auf Basis einer ersten Repräsentation vorherzusagen. Prinzipiell könnte für diesen Zweck ein Modell trainiert werden, die dritte Repräsentation direkt auf Basis der ersten Repräsentation zu erzeugen ("direktes Training"). Erfindungsgemäß wird das Modell des maschinellen Lernens jedoch trainiert, die dritte Repräsentation nicht in einem Schritt auf Basis der ersten Repräsentation zu erzeugen, sondern in zwei Schritten, wobei in einem ersten Schritt eine zweite Repräsentation vorhergesagt wird, auf deren Basis dann die dritte Repräsentation vorhergesagt wird. Der Vorteil bei dem erfindungsgemäßen iterativen Ansatz gegenüber dem genannten "direkten Trainieren" besteht unter anderem darin, dass zusätzliche Trainingsdaten (z.B. zweite Repräsentationen, die den Untersuchungsbereich in dem zweiten Zustand repräsentieren), verwendet werden können und damit eine höhere Vorhersagegenauigkeit erreicht werden kann. Ferner lernt das Modell keine Abbildung von einer Repräsentation auf eine andere (oder, wie im Fall von WO2019/074938A1, eine Abbildung von mehreren Repräsentationen auf eine Repräsentation), sondern es lernt, welchen Einfluss Kontrastmittel auf die Repräsentationen des Untersuchungsbereich ausübt, insbesondere wie sich zunehmende oder abnehmende Mengen an Kontrastmittel auf die Repräsentationen auswirken. Je mehr Mengen an Kontrastmittel die Trainingsdaten abdecken, desto genauer kann das Modell den Einfluss unterschiedlicher Mengen lernen.

Das in Fig. 2 dargestellte Verfahren ist eine bevorzugte Ausführungsform der vorliegenden Erfindung und umfasst zusammengefasst die Schritte:
- Empfangen einer ersten Repräsentation R₁ eines Untersuchungsbereichs, wobei die erste Repräsentation R₁ den Untersuchungsbereich ohne Kontrastmittel oder nach Applikation einer ersten Menge *a*₁ eines Kontrastmittels repräsentiert,
- Erzeugen einer vorhergesagten zweiten Repräsentation R₂^{*} des Untersuchungsbereichs mit Hilfe eines Modells des maschinellen Lernens zumindest anteilig auf Basis der ersten Repräsentation R₁, wobei die zweite Repräsentation R₂^{*} den Untersuchungsbereich nach Applikation einer zweiten Menge *a₂* des Kontrastmittels repräsentiert, wobei die zweite Menge vorzugsweise größer als Null und vorzugsweise größer als die erste Menge ist,
- Erzeugen einer vorhergesagten dritten Repräsentation R₃^{*} des Untersuchungsbereichs mit Hilfe des Modells des maschinellen Lernens zumindest anteilig auf Basis der vorhergesagten zweiten Repräsentation R₂^{*}, wobei die vorhergesagte dritte Repräsentation R₃^{*} den Untersuchungsbereich nach Applikation einer dritten Menge *a*₃ des Kontrastmittels repräsentiert, wobei die dritte Menge vorzugsweise größer als die zweite Menge ist,
- Ausgeben und/oder Speichern und/oder Übermitteln der dritten Repräsentation R₃^{*},
wobei das Modell des maschinellen Lernens auf Basis von Trainingsdaten trainiert wurde, wobei die Trainingsdaten eine Vielzahl von Datensätzen umfasst, wobei jeder Datensatz eine erste Referenz-Repräsentation, eine zweite Referenz-Repräsentation und eine dritte Referenz-Repräsentation umfasst, wobei die erste Referenz-Repräsentation den Untersuchungsbereich ohne Kontrastmittel oder nach der Applikation der ersten Menge des Kontrastmittels repräsentiert, die zweite Referenz-Repräsentation den Untersuchungsbereich nach der Applikation der zweiten Menge des Kontrastmittels repräsentiert und die dritte Referenz-Repräsentation den Untersuchungsbereich nach der Applikation der dritten Menge des Kontrastmittels repräsentiert, wobei das Modell des maschinellen Lernens trainiert wurde, zumindest anteilig auf Basis der ersten Referenz-Repräsentation die zweite Referenz-Repräsentation vorherzusagen und zumindest anteilig auf Basis der vorhergesagten zweiten Referenz-Repräsentation die dritte Referenz-Repräsentation zu vorherzusagen.

Allgemein kann das erfindungsgemäße Modell des maschinellen Lernens trainiert werden, ausgehend von einer ersten Referenz-Repräsentation R₁ eine Zahl *n-1* von vorhergesagten Referenz-Repräsentationen R₂^{*} bis R*ₙ*^{*} zu erzeugen, wobei *n* eine ganze Zahl größer als 2 ist, wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 1 bis *n* durchläuft, wobei die Mengen *a*₁ bis *aₙ* eine Folge von vorzugsweise zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft.

Dies sei am Beispiel von Fig. 3 näher erläutert. Fig. 3 kann als eine Erweiterung des in Fig. 2 gezeigten Schemas von drei Repräsentationen auf eine (zumindest innerhalb definierter Grenze) beliebige Zahl *n* an Repräsentationen verstanden werden, wobei *n* eine ganze Zahl größer als 2 ist. Vorzugsweise liegt die Zahl *n* im Bereich von 3 bis 100. Die Zahl *n* kann jedoch auch größer als 100 sein.

Fig. 3 zeigt ein Modell des maschinellen Lernens M. Das Modell ist dreimal aufgeführt; es handelt sich aber stets um dasselbe Modell. Das Modell des maschinellen Lernens M wird trainiert, eine Zahl *n-1* an Repräsentationen vorherzusagen. Im vorliegenden Beispiel ist *n* eine ganze Zahl größer als 3. Dem Modell wird eine erste Repräsentation R₁ als Eingabedaten zugeführt. Die erste Repräsentation R₁ repräsentiert einen Untersuchungsbereich eines Untersuchungsobjekts ohne Kontrastmittel oder nach der Applikation einer ersten Menge *a*₁ eines Kontrastmittels. Das Modell des maschinellen Lernens M ist konfiguriert, zumindest anteilig auf Basis der ersten Repräsentation R₁ und auf Basis von Modellparametern MP eine Ausgabe R₂^{*} zu erzeugen. Die Ausgabe R₂^{*} stellt eine vorhergesagte zweite Repräsentation dar, die den Untersuchungsbereich nach Applikation einer zweiten Menge *a*₂ des Kontrastmittels repräsentiert. Das Modell ist ferner konfiguriert, zumindest anteilig auf Basis der Ausgabe R₂^{*} und Modellparametern MP eine Ausgabe R₃^{*} zu erzeugen. Die Ausgabe R₃^{*} stellt eine vorhergesagte dritte Repräsentation dar, die den Untersuchungsbereich nach einer dritten Menge *a*₃ des Kontrastmittels repräsentiert. Das Modell ist ferner konfiguriert, zumindest anteilig auf Basis der Ausgabe R₃^{*} und Modellparametern MP eine weitere Ausgabe zu erzeugen, die eine vorgesagte Repräsentation des Untersuchungsbereichs nach einer Applikation einer weiteren Menge des Kontrastmittels repräsentiert. Dieses Schema wird bis zu einer Ausgabe R*ₙ*^{*} fortgesetzt. Die Ausgabe R*ₙ*^{*} stellt eine vorhergesagte n-te Repräsentation dar, die den Untersuchungsbereich nach der Applikation einer n-ten Menge *aₙ* des Kontrastmittels repräsentiert. Die Mengen an Kontrastmittel *a*₁, *a*₂, *a*₃, *... , aₙ* bilden eine Folge. Bei der Folge kann es sich beispielsweise um eine Folge zunehmender Kontrastmittelmengen handeln. Mit anderen Worten: vorzugsweise ist die zweite Menge *a*₂ größer als Null und größer als die erste Menge *a*₁, die dritte Menge *a*₃ größer als die zweite Menge *a*₂, die vierte Menge *a*₄ größer als die dritte Menge *a*₃ und so fort. Allgemein ausgedrückt: die j-te Menge *aⱼ* ist vorzugsweise größer als die (*j*-1)-te Menge *a*_{*j*-1}, wobei *j* ein Index ist der die Zahlen von 2 bis *n* durchläuft.

Das in Fig. 3 dargestellte Verfahren zum Trainieren des erfindungsgemäßen Modells des maschinellen Lernens ist ein weiterer Gegenstand der vorliegenden Erfindung und umfasst zusammengefasst die folgenden Schritte:
- Empfangen von Trainingsdaten,
   ∘ wobei die Trainingsdaten eine Vielzahl von Datensätzen einer Vielzahl von Referenz-Untersuchungsobjekten umfassen,
   ∘ wobei die Datensätze Referenz-Repräsentationen eines Untersuchungsbereichs umfassen, wobei der Untersuchungsbereich die Leber oder ein Teil der Leber eines Menschen ist, wobei die Referenz-Repräsentationen radiologische Aufnahmen sind,
   ∘ wobei jeder Datensatz eine oder mehrere Referenz-Repräsentationen einer Zahl *n* an Referenz-Repräsentationen umfasst, wobei *n* eine ganze Zahl größer als 2 ist,
   ∘ wobei jede Referenz-Repräsentation R*ᵢ* den Untersuchungsbereich nach Applikation einer Menge *a*ᵢ eines Kontrastmittels repräsentiert, wobei *i* ein Index ist, der ganze Zahlen von 1 bis *n* durchläuft, wobei die Mengen *a*₁ bis *aₙ* eine Folge von vorzugsweise zunehmenden Mengen bilden,
- Trainieren des Modells des maschinellen Lernens,
   ∘ wobei das Modell trainiert wird, ausgehend von der Referenz-Repräsentation R₁ nacheinander vorhergesagte Referenz-Repräsentationen R₂^{*} bis R*ₙ*^{*} zu erzeugen,
   ∘ wobei die Erzeugung der Referenz-Repräsentation R₂^{*} zumindest anteilig auf Basis der Referenz-Repräsentation R₁ basiert und die Erzeugung jeder nachfolgenden vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis n durchläuft,
   ∘ wobei die vorhergesagte Referenz-Repräsentation R*ₖ*^{*} den Untersuchungsbereich nach Applikation der Menge *aₖ* des Kontrastmittels repräsentiert und die vorhergesagte Referenz-Repräsentation R_{*k*-1}^{*} den Untersuchungsbereich nach Applikation der Menge *a*_{*k*-1} des Kontrastmittels repräsentiert
   ∘ wobei das Trainieren des Modells des maschinellen Lernens ferner die Schritte umfasst:
      - für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- Nutzen des trainierten Modells des maschinellen Lernens zur Vorhersage.

Im Fall des in Fig. 3 gezeigten Beispiels liegen, neben der ersten Repräsentation R₁, weitere (reale, messtechnisch erzeugte) Repräsentationen R*ⱼ* (mit *j* = 2 bis *n*) des Untersuchungsbereichs nach der Applikation der Mengen *aⱼ* (mit *j* = 2 bis *n*) des Kontrastmittels vor, die als Zieldaten (*ground truth*) zum Trainieren des Modells des maschinellen Lernens verwendet werden können. So kann die Abweichung zwischen einer Ausgabe R*ⱼ*^{*} (die eine vorgesagte j-te Repräsentation des Untersuchungsbereichs nach der Applikation einer Menge *aⱼ* des Kontrastmittels darstellt) und der Repräsentation R*ⱼ* mit einer Fehlerfunktion LF*ⱼ* quantifiziert werden: eine Fehlerfunktion LF₂ kann die Abweichung zwischen der Repräsentation R₂ und der vorhergesagten Repräsentation R₂^{*} quantifizieren, eine Fehlerfunktion LF₃ kann die Abweichung zwischen der Repräsentation R₃ und der vorhergesagten Repräsentation R₃^{*} quantifizieren, und so fort.

In einem Ende-zu-Ende-Training kann eine Gesamtfehlerfunktion LF verwendet werden, die alle einzelnen Abweichungen berücksichtigt; dabei kann es sich beispielsweise um die Summe der einzelnen Abweichungen handeln: $LF = {LF}_{2} + {LF}_{3} + … + {LF}_{n} = {\sum }_{j = 2}^{n} {LF}_{j}$

Es kann sich aber auch um eine gewichtete Summe handeln: $LF = {w}_{2} ⋅ {LF}_{2} + {w}_{3} ⋅ {LF}_{3} + … + {w}_{n} ⋅ {LF}_{n} = {\sum }_{j = 2}^{n} {w}_{j} ⋅ {LF}_{j}$ wobei die Gewichtsfaktoren w*ᵢ* beispielsweise Werte von 0 bis 1 annehmen können.

Die Gewichtung hat den Vorteil, dass den Repräsentationen, die dem Modell des maschinellen Lernens zugeführt werden, unterschiedliches Gewicht bei der Erzeugung einer vorhergesagten Repräsentation beigemessen wird.

So ist es beispielweise möglich, dass die Gewichtsfaktoren w*ⱼ* in der Reihe w₂, w₃, ..., w*ₙ* mit der Menge an Kontrastmittel *a*₂, *a*₃, *..., aₙ* zunehmen und damit Repräsentationen, deren Menge an Kontrastmittel näher an der Menge *aₙ* liegt, höher gewichtet werden. Dabei kann die Zunahme logarithmisch, linear, quadratisch, kubisch oder exponentiell sein oder eine andere Zunahme sein. Es ist auch denkbar, den Repräsentationen mit einer größeren Menge an Kontrastmittel weniger Gewicht beizumessen, indem die Gewichtsfaktoren in der Reihe w₂, w₃, ..., w*ₙ* mit zunehmender Menge an Kontrastmittel *a*₂, *a*₃, ..., *aₙ* abnehmen und damit Repräsentationen, die näher an der initialen Repräsentation R₁ liegen, mehr Gewicht beimessen. Auch eine solche Abnahme kann logarithmisch, linear, quadratisch, kubisch oder exponentiell sein oder eine andere Abnahme sein.

Es ist auch denkbar, dass die Trainingsdaten unvollständige Datensätze umfassen. Das bedeutet, dass einige Datensätze von Untersuchungsobjekten nicht alle Referenz-Repräsentationen R₁ bis R*ₙ* umfassen. Wie weiter hinten in der Beschreibung erläutert, können auch unvollständige Datensätze zum Trainieren des Modells des maschinellen Lernens verwendet werden. Falls eine Referenz-Repräsentation R*ⱼ* fehlt, kann der Gewichtsfaktor w*ⱼ*, der die Fehlerfunktion zu der Referenz-Repräsentation R*ⱼ* und der vorhergesagten Referenz-Repräsentation R*ⱼ*^{*} gewichten soll, auf Null gesetzt werden, wobei *j* eine ganze Zahl ist, die die Werte 2 bis *n* annehmen kann.

Es ist auch denkbar, dass bei dem Training zufällige Zahlen 1 ≤ *j < k* ≤ *n* und die damit verbundenen Teilfolgen von Kontrastmittelmengen *aⱼ, a*_{*j*+1}, *..., a*_{*k-*1}, *aₖ* betrachtet werden. Das oben beschriebene Lernproblem kann dann auf diesen (optional von Zeit zu Zeit variierenden) Teilfolgen gelöst werden. Zum Beispiel ist denkbar, dass eine zufällige initiale Kontrastmittelmenge *aⱼ* (1 ≤ *j* ≤ *n*-2) bestimmt wird und das Modell auf dessen Basis stets die Repräsentationen der nachfolgenden zwei Kontrastmittelmenge *a*_{*j*+1}, *a*_{*j*+2} synthetisieren soll.

In Fig. 3 ist auch schematisch dargestellt, dass zur Erzeugung einer Repräsentation R*ⱼ*^{*}, die den Untersuchungsbereich nach der Applikation einer Menge *aⱼ* des Kontrastmittels repräsentiert, neben der Repräsentation R_{*j*-1}^{*}, die den Untersuchungsbereich nach der Applikation einer Menge *a*_{*j*-1} repräsentiert, auch weitere Repräsentation R_{*j*-2}^{*} und/oder R_{*j-*3}^{*} und/oder ... bis R₁, die den Untersuchungsbereich nach der Applikation weiteren Mengen *a*_{*j*-2}, *a*_{*j*-3} ... bis *a*₁ repräsentieren, verwendet werden können, wobei *j* eine ganze Zahl ist, die die Werte 2 bis *n* annehmen kann. Dies wird durch die gestrichelten Pfeile zum Ausdruck gebracht. So kann zur Vorhersage der dritten Repräsentation neben der vorhergesagten zweiten Repräsentation auch die erste Repräsentation dem Modell des maschinellen Lernens zugeführt werden. Zur Vorhersage einer vierten Repräsentation kann neben der vorhergesagten dritten Repräsentation auch die erste Repräsentation und/oder die zweite Repräsentation dem Modell des maschinellen Lernens zugeführt werden.

Es können zur Vorhersage von Repräsentationen auch weitere Informationen in das Modell des maschinellen Lernens einfließen, wie beispielsweise Informationen über die Menge an Kontrastmittel, die jeweils appliziert worden ist. Mit anderen Worten: neben der ersten Repräsentation können zur Vorhersage der zweiten Repräsentation auch Informationen zu der Menge an appliziertem Kontrastmittel, die durch die erste Repräsentation repräsentiert wird, verwendet werden. Auch die vorhergesagte dritte Repräsentation kann auf Basis der vorhergesagten zweiten Repräsentation und Informationen zu der zweiten Menge an Kontrastmittel erzeugt werden. Wird eine Informationen über die Menge an Kontrastmittel einer Repräsentation mitgegeben, so "weiß" das Modell des maschinellen Lernens, an welcher Stelle der Folge von Kontrastmittelmengen es sich jeweils befindet.

Weitere Informationen, die zum Trainieren und zur späteren Vorhersage des Modells des maschinellen Lernens verwendet werden können, sind z.B. Informationen zum Untersuchungsbereich, zum Untersuchungsobjekt, zu Bedingungen, die bei der Erzeugung der Repräsentation herrschten, Informationen zu dem Kontrastmittel, Informationen zu der Kontrastmittelmenge, die dem Untersuchungsobjekt appliziert worden ist, und andere/weitere.

Das erfindungsgemäße Modell des maschinellen Lernens kann als eine Transformation verstanden werden, die auf eine Repräsentation eines Untersuchungsbereichs in einem Zustand einer Folge von Zuständen angewendet werden kann, um eine Repräsentation des Untersuchungsbereichs in einem nachfolgenden Zustand der Folge von Zuständen vorherzusagen. Jeder Zustand wird durch eine Menge an appliziertem Kontrastmittel repräsentiert. Die Transformation kann einfach (einmal) angewendet werden, um eine Repräsentation des Untersuchungsbereichs in dem unmittelbar nachfolgenden Zustand vorherzusagen, oder mehrfach (mehrmals) angewendet werden, um eine Repräsentation des Untersuchungsbereichs in einem in der Folge der Zustände weiter hinten liegenden Zustand vorherzusagen.

Liegt eine Folge von *n* Zuständen Z₁ bis Z*ₙ* vor, und liegt zu jedem Zustand Z*ᵢ* eine Repräsentation R*ᵢ* vor, die den Untersuchungsbereich in dem Zustand Z*ᵢ* repräsentiert, so kann das Modell M des maschinellen Lernens ausgehend von der Repräsentation R₁ q-fach angewendet werden, um eine Repräsentation R_{1+*q*} zu erzeugen, die den Untersuchungsbereich in dem Zustand Z_{1-*q*} repräsentiert, wobei q die Werte 1 bis *n*-1 annehmen kann: ${M}^{q} \left({R}_{1}\right) = {{R}_{1 + q}}^{∗}$ $q = 1 : M \left({R}_{1}\right) = {{R}_{2}}^{∗}$ $q = 2 : M \left({{R}_{2}}^{∗}\right) = M \left(M\left({R}_{1}\right)\right) = {M}^{2} \left({R}_{1}\right) = {{R}_{3}}^{∗}$ $q = 3 : M \left({{R}_{3}}^{∗}\right) = M \left(M\left({{R}_{2}}^{∗}\right)\right) = M \left(M\left(M\left({R}_{1}\right)\right)\right) = {M}^{3} \left({R}_{1}\right) = {{R}_{4}}^{∗}$ $q = n - 1 : M \left({{R}_{n - 1}}^{∗}\right) = M \left(M\left({{R}_{n - 2}}^{∗}\right)\right) = … = {M}^{n - 1} \left({R}_{1}\right) = {{R}_{n}}^{∗}$

Der jeweilige Zustand gibt an, welche Kontrastmittelmenge appliziert worden ist. Eine Folge von Zuständen kann also beispielsweise den Untersuchungsbereich nach Applikation einer Folge von zunehmenden Kontrastmittelmengen charakterisieren.

Eine Fehlerfunktion, die alle Abweichungen zwischen vorhergesagten Repräsentationen R*_{q}*^{*} und realen (messtechnisch erzeugten) Repräsentationen R*_{q}* quantifiziert, kann beispielsweise durch die folgende Formel ausgedrückt werden: $LV = {w}_{2} ⋅ d \left(M\left({R}_{1}\right), {R}_{2}\right) + {w}_{3} ⋅ d \left({M}^{2}\left({R}_{1}\right), {R}_{3}\right) + … + {w}_{n} ⋅ d \left({M}^{n - 1}\left({R}_{1}\right), {R}_{n}\right)$

Dabei ist LV der Fehlerwert, der sich für einen Datensatz umfassend die Referenz-Repräsentationen R₁, R₂, ..., R*ₙ* ergibt. **d** ist eine Fehlerfunktion, die die Abweichungen zwischen einer vorhergesagten Repräsentation **M**(R_{*q*-1}) und der Referenz-Repräsentation R*_{q}* quantifiziert. Wie bereits weiter vorne in der Beschreibung beschrieben, kann es sich dabei beispielsweise um eine der folgenden Fehlerfunktionen handeln: L1 loss, L2 loss, Lp loss, Strukturähnlichkeitsindexmaß (SSIM), *VGG loss, perceptual loss* oder eine Kombination davon.

w₂, w₃, ..., w*ₙ* sind Gewichtsfaktoren, die ebenfalls weiter vorne in der Beschreibung bereits beschrieben wurden.

*n* gibt die Zahl der Zustände (die Zahl der unterschiedlichen Mengen an Kontrastmittel) an.

Es ist auch denkbar, dass der Fehlerwert die maximale Abweichung ist, die für einen Datensatz berechnet wird: $LV = max \left({w}_{2}⋅d\left(M\left({R}_{1}\right), {R}_{2}\right), {w}_{3}⋅d\left({M}^{2}\left({R}_{1}\right), {R}_{3}\right),…, {w}_{n}⋅d\left({M}^{n - 1}\left({R}_{1}\right), {R}_{n}\right)\right)$ wobei auch in dieser Formel durch die Gewichtsfaktoren den einzelnen Abweichungen ein unterschiedliches Gewicht beigemessen werden kann.

Wie bereits weiter vorne in der Beschreibung angedeutet, sei angemerkt, dass es zum Trainieren des Modells des maschinellen Lernens nicht erforderlich ist, dass jeder Datensatz der Trainingsdaten Repräsentationen von allen Kontrastmittelmengen, die das Modell lernen soll, beinhaltet. Dies sei an einem Beispiel erläutert. Es sei angenommen, dass das Modell des maschinellen Lernens trainiert werden soll, Repräsentationen eines Untersuchungsbereichs mit den zunehmenden Kontrastmittelmengen *a*₁ bis *a*₆ vorherzusagen. Es sei angenommen, dass zum Trainieren des Modells des maschinellen Lernens Trainingsdaten ausreichend sind, die 10 Datensätze von 10 Untersuchungsobjekten umfassen. Jeder Datensatz umfasst Repräsentationen des Untersuchungsbereichs nach Applikation verschiedener Mengen an Kontrastmittel, z.B.:
Datensatz 1: R₁, R₃, R₄, R₅, R₆
Datensatz 2: R₁, R₂, R₄, R₆
Datensatz 3: R₁, R₂, R₃, R₄, R₅
Datensatz 4: R₁, R₂, R₃, R₅, R₆
Datensatz 5: R₂, R₃, R₅, R₆
Datensatz 6: R₂, R₃, R₄, R₅, R₆
Datensatz 7: R₂, R₃, R₅, R₆
Datensatz 8: R₃, R₅, R₆
Datensatz 9: R₃, R₄, R₅, R₆
Datensatz 10: R₃, R₄, R₆

In dem Beispiel gibt es keinen einzigen "vollständigen" Datensatz, also keinen Datensatz, der alle möglichen Repräsentationen R₁, R₂, R₃, R₄, R₅ und R₆ umfasst. Dennoch ist es möglich, das Modell des maschinellen Lernens auf Basis von solchen Trainingsdaten zu trainieren, eine Repräsentation des Untersuchungsbereichs nach Applikation einer jeden Menge *a*₂ bis *a*₆ vorherzusagen. Dies ist ein Vorteil der vorliegenden Erfindung gegenüber dem oben beschriebenen "direkten Trainieren".

Ist das Modell des maschinellen Lernens trainiert, können dem Modell neue (d.h. nicht im Training verwendete) Repräsentationen eines Untersuchungsbereichs nach Applikation einer Menge des Kontrastmittels zugeführt werden, und das Modell kann eine oder mehrere Repräsentationen des Untersuchungsbereichs nach Applikation einer anderen (größeren) Menge vorhersagen (erzeugen).

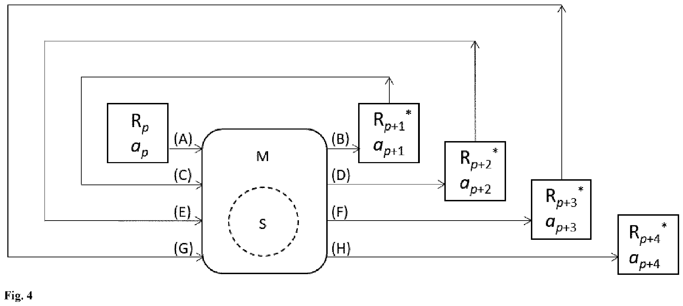

Dies ist beispielhaft und schematisch in Fig. 4 dargestellt. In dem in Fig. 4 dargestellten Beispiel werden ausgehend von einer Repräsentation R*ₚ*, die den Untersuchungsbereich nach der Applikation der Menge *aₚ* eines Kontrastmittels repräsentiert, eine Folge von Repräsentationen R_{*p*+1}^{*}, R_{*p+*2}^{*}, R_{*p*+3}^{*} und R_{*p*+4}^{*} erzeugt, die den Untersuchungsbereich nach der Applikation der Mengen *a*_{*p*+1}, *a*_{*p*+2}, *a*_{*p*+3} und *a*_{*p*+4} repräsentieren. Die Kontrastmittelmengen *a*_{*p*+1}, *a*_{*p*+2}, *a*_{*p*+3} und *a*_{*p*+4} bilden vorzugsweise eine Folge von zunehmenden Mengen: *a*_{*p*+1} *< a*_{*p*+2} *< a*_{*p*+3} *< a*_{*p*+4}*.*

In Schritt (A) wird dem Modell des maschinellen Lernens M die Repräsentation R*ₚ* zugeführt. Neben der Repräsentation R*ₚ* können dem Modell des maschinellen Lernens M auch Informationen zu der Kontrastmittelmenge *aₚ* und/oder weitere/andere Informationen zugeführt werden, wie weiter vorne in dieser Beschreibung beschrieben.

Das Modell des maschinellen Lernens M erzeugt in Schritt (B) auf Basis der zugeführten Eingabedaten die Repräsentation R_{*p*+1}^{*}, die den Untersuchungsbereich nach der Applikation der Menge *a*_{*p+*1} repräsentiert.

In Schritt (C) wird dem Modell des maschinellen Lernens M die zuvor erzeugte Repräsentation R_{*p*+1}^{*} zugeführt. Neben der Repräsentation R_{*p*+1}^{*} können dem Modell des maschinellen Lernens M auch Informationen zu der Kontrastmittelmenge *a*_{*p*+1} und/oder weitere Informationen zugeführt werden. Ferner können dem Modell des maschinellen Lernens auch die Repräsentation R*ₚ* und/oder Informationen zu der Kontrastmittelmenge *aₚ* zugeführt werden.

Vorzugsweise umfasst das Modell des maschinellen Lernens M einen Speicher S, der Eingabedaten (und vorzugsweise auch Ausgabedaten) speichert, so dass einmal eingegebene Eingabedaten und/oder erzeugte Ausgabedaten nicht erneut empfangen und/oder eingegeben werden müssen, sondern dem Modell des maschinellen Lernens bereits zur Verfügung stehen. Dies gilt nicht nur für die in diesem Beispiel beschriebene Nutzung des trainierten Modells des maschinellen Lernens zur Vorhersage, sondern auch für das Trainieren des erfindungsgemäßen Modells des maschinellen Lernens.

Das Modell des maschinellen Lernens M erzeugt in Schritt (D) auf Basis der zugeführten Eingabedaten die Repräsentation R_{*p*+2}^{*}, die den Untersuchungsbereich nach der Applikation der Kontrastmittelmenge *a*_{*p*+2} repräsentiert.

In Schritt (E) wird dem Modell des maschinellen Lernens M die zuvor erzeugte Repräsentation R_{*p*+2}^{*} zugeführt. Neben der Repräsentation R_{*p*+2}^{*} können dem Modell des maschinellen Lernens M auch Informationen zu der Kontrastmittelmenge *a*_{*p*+2} und/oder weitere Informationen zugeführt werden. Ferner können dem Modell des maschinellen Lernens auch die Repräsentation R*ₚ* und/oder die Repräsentation R_{*p*+1}^{*} und/oder Informationen zu den Kontrastmittelmengen *aₚ* und/oder *a*_{*p*+1} zugeführt werden.

Das Modell des maschinellen Lernens M erzeugt in Schritt (F) auf Basis der zugeführten Eingabedaten die Repräsentation R_{*p*+3}^{*}, die den Untersuchungsbereich nach der Applikation der Kontrastmittelmenge *a*_{*p*+3} repräsentiert.

In Schritt (G) wird dem Modell des maschinellen Lernens M die zuvor erzeugte Repräsentation R_{*p*+3}^{*} zugeführt. Neben der Repräsentation R_{*p*+3}^{*} können dem Modell des maschinellen Lernens M auch Informationen zu der Kontrastmittelmenge *a*_{*p*+3} und/oder weitere Informationen zugeführt werden. Ferner können dem Modell des maschinellen Lernens auch die Repräsentation R*ₚ* und/oder die Repräsentation R_{*p*+1}^{*} und/oder die Repräsentation R_{*p*+2}^{*} und/oder Informationen zu den Kontrastmittelmengen *aₚ* und/oder *a*_{*p*+1} und/oder *a*_{*p*+2} zugeführt werden.

Das Modell des maschinellen Lernens M erzeugt in Schritt (H) auf Basis der zugeführten Eingabedaten die Repräsentation R_{*p*+4}^{*}, die den Untersuchungsbereich nach der Applikation der Kontrastmittelmenge *a*_{*p*+4} repräsentiert.

Die erzeugten Repräsentationen R_{*p*+1}^{*}, R_{*p+*2}^{*}, R_{*p*+3}^{*} und/oder R_{*p*+4}^{*} können ausgegeben (z.B. auf einem Monitor angezeigt und/oder mit einem Drucker ausgedruckt) und/oder in einem Datenspeicher gespeichert und/oder an ein (separates) Computersystem übermittelt werden.

Wurde das erfindungsgemäße Modell des maschinellen Lernens trainiert, ausgehend von einer ersten Repräsentation R₁, die den Untersuchungsbereich nach der Applikation einer ersten Menge *a*₁ des Kontrastmittels repräsentiert, eine Folge von vorhergesagten Repräsentationen R₁^{*}, R₂^{*}, ..., R*ₙ*^{*} zu erzeugen, wobei jede vorhergesagte Repräsentation R*ⱼ*^{*} den Untersuchungsbereich nach der Applikation einer Menge *aⱼ* des Kontrastmittels repräsentiert, wobei *j* ein Index ist, der ganze Zahlen von 2 bis *n* durchläuft, dann kann einem solchen Modell eine neue Repräsentation R*ₚ* zugeführt werden, die den Untersuchungsbereich nach der Applikation der Menge *aₚ* des Kontrastmittels repräsentiert, und das Modell des maschinellen Lernens kann die vorhergesagten Repräsentationen R_{*p*+1}^{*}, R_{*p+*2}^{*}, ... , R*ₙ*^{*} erzeugen, wobei *p* eine Zahl ist, die die Werte 2 bis *n* annehmen kann.

Das bedeutet, dass dem trainierten Modell nicht zwangsläufig eine neue Repräsentation R₁, die den Untersuchungsbereich nach der Applikation der ersten Menge *a*₁ repräsentiert, zugeführt werden muss, und das trainierte Modell des maschinellen Lernens ist auch nicht darauf beschränkt, nur die Repräsentation R*ₙ*, die den Untersuchungsbereich nach der Applikation der (vorzugsweise größten) Menge *aₙ* repräsentiert, vorherzusagen. Stattdessen kann man in der Folge von Kontrastmittelmengen *a*₁ bis *aₙ* an jeder Stelle "einsteigen" und "aussteigen", d.h. auf Basis einer beliebigen Repräsentation zu der Folge von Kontrastmittelmengen eine beliebige andere Repräsentation zu einer in der Folge der Konzentrationsmengen nachfolgenden Konzentrationsmenge vorhersagen.

Ist also ein Modell des maschinellen Lernens beispielsweise trainiert worden, den Effekt einer zunehmenden Menge an Kontrastmittel in einer Folge zunehmender Kontrastmittelmengen *a*₁, *a*₂, *a*₃, *a*₄ zu lernen, dann kann dem trainierten Modell des maschinellen Lernens beispielsweise
- eine Repräsentation R₁ zugeführt werden, um eine Repräsentation R₂ und/oder R₃ und/oder R₄ zu erzeugen, oder
- eine Repräsentation R₂ zugeführt werden, um eine Repräsentation R₃ und/oder R₄ zu erzeugen, oder
- eine Repräsentation R₃ zugeführt werden, um eine Repräsentation R₄ zu erzeugen,
wobei die Repräsentation R₁ den Untersuchungsbereich nach der Applikation der Menge *a*₁ repräsentiert, die Repräsentation R₂ den Untersuchungsbereich nach der Applikation der Menge *a*₂ repräsentiert, die Repräsentation R₃ den Untersuchungsbereich nach der Applikation der Menge *a*₃ repräsentiert und die Repräsentation R₄ den Untersuchungsbereich nach der Applikation der Menge *a*₄ repräsentiert.

Es ist sogar möglich, Repräsentationen zu erzeugen, die den Untersuchungsbereich nach Applikation einer Kontrastmittelmenge repräsentieren, die gar nicht Gegenstand des Trainings gewesen ist. Anstatt also beim der Repräsentation R*ₙ* (z.B. R₄) zu stoppen, können auch eine Repräsentation R_{*n*+1} (z.B. R₅), R_{*n*+2} (z.B. R₆) und so weiter erzeugt werden. Das trainierte Modell des maschinellen Lernens kann also verwendet werden, um den gelernten Effekt einer vorzugsweise zunehmenden Kontrastmittelmenge fortzusetzen und Repräsentationen zu berechnen, die messtechnisch niemals erzeugt worden sind. Insofern kann das trainierte Modell des maschinellen Lernens zur Extrapolation auf sehr hohe Kontrastmittelmengen verwendet werden, die man einem Untersuchungsobjekt niemals applizieren würde.

Ferner sind die Vorhersagen nicht auf zunehmende Kontrastmittelmengen beschränkt. Es ist auch möglich Repräsentationen mit abnehmenden Kontrastmittelmengen vorherzusagen. Zum einen kann das Modell des maschinellen Lernens grundsätzlich in beide Richtungen trainiert werden: in Richtung zunehmender Kontrastmittelmengen und in Richtung abnehmender Kontrastmittelmengen. Zum anderen führt das Modell des maschinellen Lernens an einer eingegebenen Repräsentation eine Transformation aus, die sich prinzipiell auch umkehren lässt. Aus der Analyse der mathematischen Funktionen des Modells, die eine Eingabe-Repräsentation in eine Ausgabe-Repräsentation transformieren, lassen sich Umkehrfunktionen ermitteln, die den Prozess umkehren und die vorherige Ausgabe-Repräsentation wieder in die vorherige Eingabe-Repräsentation zurückverwandelt. Die Umkehrfunktionen können dann verwendet werden, Repräsentationen mit abnehmenden Kontrastmittelmengen vorherzusagen, auch wenn das Modell trainiert worden ist, Repräsentationen mit zunehmenden Kontrastmittelmengen vorherzusagen und umgekehrt.

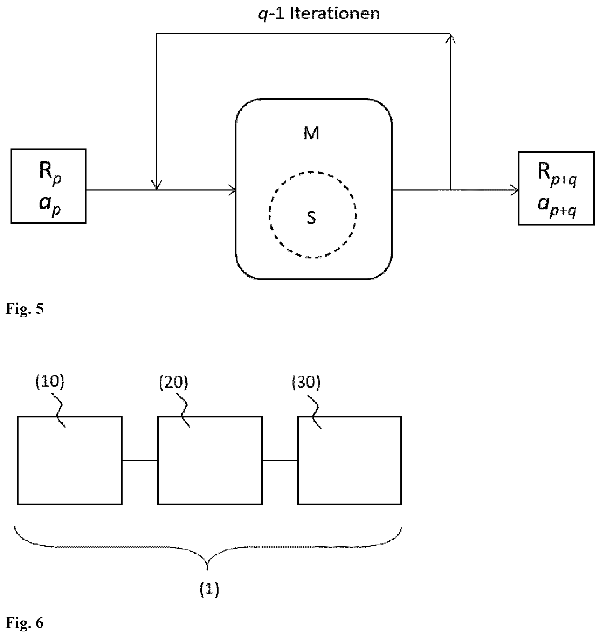

Fig. 5 zeigt eine Erweiterung des in Fig. 4 gezeigten Schemas. Während in Fig. 4 auf Basis einer Repräsentation R*ₚ*, die den Untersuchungsbereich nach der Applikation einer Kontrastmittelmenge *a*ₚ repräsentiert, nacheinander die Repräsentationen R_{*p*+1}^{*}, R_{*p+*2}^{*}, R_{*p*+3}^{*} und R_{*p*+4}^{*} erzeugt werden, die den Untersuchungsbereich nach der Applikation der Mengen *a*_{*p*+1}, *a*_{*p*+2}, *a*_{*p*+3} und *a*_{*p*+4} des Kontrastmittels repräsentieren, ist in Fig. 5 gezeigt, wie auf Basis der Repräsentation R*ₚ*, die den Untersuchungsbereich nach der Applikation der Kontrastmittelmenge *aₚ* repräsentiert, nacheinander die Repräsentationen R_{*p*+1}^{*} bis R*_{p+q}*^{*} erzeugt werden, wobei *p* eine ganze Zahl ist, die die Werte 2 bis *n* annehmen kann, und q eine ganze Zahl größer als 1 ist. In Fig. 5 kommt der iterative Charakter des Modells des maschinellen Lernens M besonders deutlich zum Ausdruck. Das Modell des maschinellen Lernens M wird, ausgehend von der Repräsentation R*ₚ q*-mal angewandt, wobei (*q*-1)-mal die Ausgabedaten wieder zurück in das Modell des maschinellen geschleust werden.

Es sei darauf hingewiesen, dass das (trainierte oder untrainierte) Modell des maschinellen Lernens nicht auf eine komplette radiologische Aufnahme (z.B. eine MRT-Aufnahme oder einen CT-Scan oder dergleichen) angewendet werden muss. Es ist möglich, das Modell des maschinellen Lernens nur auf einen Teil einer radiologischen Aufnahme anzuwenden. Es ist beispielsweise möglich, eine radiologische Aufnahme zunächst zu segmentieren, um einen Bereich von Interesse (engl. *region of interest*) zu identifizieren/selektieren. Das Modell kann dann beispielsweise ausschließlich auf den Bereich von Interesse angewandt werden.

Ferner kann die Anwendung des Modells des maschinellen Lernens einen oder mehrere Vorverarbeitungs- und/oder Nachverarbeitungsschritte umfassen. Es Beispiel denkbar, eine empfangene Repräsentation des Untersuchungsbereichs zunächst einer oder mehreren Transformationen zu unterziehen, wie beispielsweise einer Bewegungskorrektur, einer Farbraumumwandlung, einer Normalisierung, einer Segmentierung und/oder dergleichen. In einem weiteren Schritt kann die transformierte Repräsentation dem Modell des maschinellen Lernens zugeführt werden, das dann eine Reihe von Iterationen (Zyklen) durchläuft, um ausgehend von der transformierten Repräsentation eine Reihe von weiteren (nachfolgenden) Repräsentationen des Untersuchungsbereich in einer Reihe von weiteren (nachfolgenden) Zuständen zu erzeugen.

Bei dem erfindungsgemäßen Modell des maschinellen Lernens kann es sich beispielsweise um ein künstliches neuronales Netzwerk handeln, oder es kann ein solches umfassen.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Repräsentationen. Üblicherweise gibt es ein Eingangsneuron für jedes Pixel oder Voxel einer Repräsentation, wenn es sich bei der Repräsentation um eine Ortsraumdarstellung in Form einer Rastergrafik handelt, oder ein Eingangsneuron für jede Frequenz, die in der Repräsentation vorhanden ist, falls es sich bei der Repräsentation um eine Frequenzraumdarstellung handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Repräsentation herrschten, Informationen zu dem Kontrastmittel, Informationen zu der Kontrastmittelmenge, die dem Untersuchungsobjekt appliziert worden ist, und andere/weitere) vorhanden sein.

Die Ausgangsneuronen können dazu dienen, eine vorhergesagte Repräsentation auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der Dynamik des Untersuchungsbereichs von einem Zustand über mindestens einen Zwischenzustand zu einem Endzustand. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich des Effekts einer zunehmenden oder abnehmenden Kontrastmittelmenge auf den Untersuchungsbereich, die verwendet werden kann, um, auf Basis einer ersten Repräsentation, die den Untersuchungsbereich nach der Applikation einer ersten Menge des Kontrastmittels repräsentiert, eine oder mehrere Repräsentationen, die den Untersuchungsbereich nach der Applikation einer anderen Menge des Kontrastmittels repräsentiert, vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann ein rekurrentes neuronales Netzwerk sein oder ein solches umfassen. Als rekurrente bzw. rückgekoppelte neuronale Netzwerke bezeichnet man neuronale Netzwerke, die sich im Gegensatz zu den Feedforward-Netzen durch Verbindungen von Neuronen einer Schicht zu Neuronen derselben oder einer vorangegangenen Schicht auszeichnen. Das künstliche neuronale Netzwerk kann beispielsweise ein *Long short-term memory* (LSTM) umfassen (siehe z.B. Y. Gao et al.: Fully convolutional structured LSTM networks for joint 4D medical image segmentation, DOI: 10.1109/ISBI.2018.8363764).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Die vorliegende Erfindung kann beispielsweise zur Reduktion der Menge an Kontrastmittel in einer radiologischen Untersuchung verwendet werden.

Für jedes Kontrastmittel gibt es eine empfohlene Menge, die einem Untersuchungsobjekt für eine definierten Zweck verabreicht kann. Üblicherweise haben Kontrastmittel eine Zulassung für einen definierten Zweck, wobei die Zulassung eine Information über die zu verabreichende (zu applizierende) Menge umfasst. Eine von einem Hersteller und/oder Vertreiber eines Kontrastmittels empfohlene Menge oder eine im Rahmen einer Zulassung vorgeschriebene Menge eines Kontrastmittels wird in dieser Beschreibung als Standardmenge bezeichnet.

So beträgt die Standardmenge von Gd-EOB-DTPA Dinatrium beispielsweise 0,025 mmol/kg Körpergewicht.

Die vorliegende Erfindung kann beispielsweise verwendet werden, um auf Basis einer Repräsentation, die einen Untersuchungsbereich nach der Applikation einer geringeren Menge eines Kontrastmittels als der Standardmenge des Kontrastmittels, eine Repräsentation des Untersuchungsbereich nach der Applikation einer Standardmenge vorherzusagen.

Dazu kann ein Modell des maschinellen Lernens auf Basis von Trainingsdaten trainiert werden, den Einfluss einer zunehmenden Menge an Kontrastmittel auf die Repräsentation des Untersuchungsbereichs zu lernen.

Der Trainingsdatensatz kann beispielsweise, neben einer Referenz-Repräsentation, die den Untersuchungsbereich nach der Applikation der Standardmenge des Kontrastmittels repräsentiert, eine Mehrzahl an Referenz-Repräsentationen umfassen, die den Untersuchungsbereich nach der Applikation unterschiedlicher Mengen an Konzentrationsmittel repräsentieren, wobei die Mengen üblicherweise kleiner sind als die Standardmenge. Es ist zum Beispiel möglich, dass die Trainingsdaten, für jedes Untersuchungsobjekt einer Vielzahl an Untersuchungsobjekten, eine oder mehrere von sechs Referenz-Repräsentationen umfassen, eine erste Referenz-Repräsentation, eine zweite Referenz-Repräsentation, eine dritte Referenz-Repräsentation, eine vierte Referenz-Repräsentation, eine fünfte Referenz-Repräsentation und eine sechste Referenz-Repräsentation.

Die erste Referenz-Repräsentation kann den Untersuchungsbereich ohne Kontrastmittel repräsentieren. Die zweite Referenz-Repräsentation kann den Untersuchungsbereich nach der Applikation von z.B. 20% der Standarmenge des Kontrastmittels repräsentieren. Die dritte Referenz-Repräsentation kann den Untersuchungsbereich nach der Applikation von z.B. 40% der Standarmenge des Kontrastmittels repräsentieren. Die vierte Referenz-Repräsentation kann den Untersuchungsbereich nach der Applikation von z.B. 60% der Standarmenge des Kontrastmittels repräsentieren. Die fünfte Referenz-Repräsentation kann den Untersuchungsbereich nach der Applikation von z.B. 80% der Standarmenge des Kontrastmittels repräsentieren. Die sechste Referenz-Repräsentation kann den Untersuchungsbereich nach der Applikation von z.B. 100% der Standarmenge des Kontrastmittels repräsentieren.

Das Modell des maschinellen Lernens kann auf Basis der Trainingsdaten trainiert, werden ausgehend von einer Referenz-Repräsentation, die den Untersuchungsbereich nach der Applikation einer Menge des Kontrastmittels (die auch Null sein kann), durch ein- oder mehrfaches Anwenden des Modells des maschinellen Lernens eine andere Referenz-Repräsentation vorherzusagen, die den Untersuchungsbereich nach der Applikation einer anderen Menge des Kontrastmittels repräsentiert.

Ist das Modell trainiert, so kann es zur Vorhersage genutzt werden. Dazu kann dem trainierten Modell des maschinellen Lernens eine Repräsentation des Untersuchungsbereichs (eines anderen, nicht im Rahmen des Trainings betrachteten Untersuchungsobjekts) zugeführt werden, die den Untersuchungsbereichs nach der Applikation einer Menge des Kontrastmittels repräsentiert, die 0% oder 20% oder 40% oder 60% oder 80% der Standardmenge des Kontrastmittels repräsentiert und mit Hilfe des Modells kann durch fünffaches (im Fall von 0%), vierfaches (im Fall von 20%), dreifaches (im Fall von 40%), zweifaches (im Fall von 60%) oder einfaches (im Fall von 80%) Anwenden des trainierten Modells des maschinellen Lernens eine Repräsentation des Untersuchungsbereichs vorhersagen, die den Untersuchungsbereich nach der Applikation der Standarmenge des Kontrastmittels repräsentiert.

In einem anderen Anwendungsbeispiel kann die vorliegende Erfindung verwendet werden, um eine Repräsentation vorherzsagen, die den Untersuchungsbereich nach der Applikation einer größeren Menge als der Standardmenge repräsentiert, ohne dass in dem Training eine so große Menge einem Untersuchungsobjekt appliziert werden muss.

Um bei dem zuvor genannten Beispiel zu bleiben, kann das trainierte Modell des maschinellen Lernens wiederholt angewendet werden, um eine Repräsentation des Untersuchungsbereichs vorherzusagen, die den Untersuchungsbereich nach der Applikation von 120% oder 140% oder 160% oder 180% oder 200% der Standardmenge repräsentiert. Auch Werte jenseits von 200% sind möglich. Auch wenn eine Verifikation, dass eine vorhergesagte Repräsentation im Realfall tatsächlich so aussehen würde, ausbleiben würde, weil eine Applikation so großer Mengen aus ethischen Gründen nicht vertretbar sein könnte, kann eine solche vorhergesagte Repräsentation einen Nutzen haben, wenn ein Radiologe in solchen Aufnahmen Funktionsstörungen und/oder Läsionen und/oder dergleichen erkennt, die er in anderen Aufnahmen nicht erkennen oder weniger sicher erkennen würde.

In einer besonders bevorzugten Ausführungsform wird die vorliegende Erfindung genutzt, um eine künstliche Kontrastverstärkung in einer CT-Aufnahme zu erzielen, die unter Verwendung eines MR-Kontrastmittels erzeugt worden ist.

MR-Kontrastmittel können grundsätzlich auch in der Computertomographie verwendet werden; allerdings ist die Kontrastverstärkung in der Computertomographie geringer als in der Magnetresonanztomographie, da der Kontrast von MR-Kontrastmitteln in der MRT auf einer anderen physikalischen Wirkung beruht als der Kontrast von MR-Kontrastmitteln in der CT. MR-Kontrastmittel führen zudem in der CT zu einer geringeren Kontrastverstärkung als übliche CT-Kontrastmittel, da sie eine geringere Röntgenabsorption aufweisen. Trotzdem kann es von Interesse sein, ein MR-Kontrastmittel in der CT einzusetzen, da es MR-Kontrastmittel gibt, die spezifisch von bestimmten Körperzellen aufgenommen werden oder spezifisch an bestimmte Körperzellen binden, während es keine vergleichbaren CT-Kontrastmittel gibt. Durch den Einsatz eines MR-Kontrastmittels in der CT können also Dinge sichtbar gemacht werden, die vorher in der CT nicht oder nicht sicher erkennbar waren.

Für die Magnetresonanztomographie stehen beispielsweise intrazelluläre Kontrastmittel zur Verfügung, die spezifisch von bestimmten Körperzellen aufgenommen werden. Als Beispiel sei das Dinatriumsalz der Gadoxetsäure (Gd-EOB-DTPA Dinatrium) genannt, das in dem US-Patent No. 6,039,931A beschrieben ist unter dem Markennamen Primovist^{®} und Eovist^{®} kommerziell erhältlich ist. Es handelt sich bei diesem Kontrastmittel um ein so genanntes hepatobiliäres Kontrastmittel, das spezifisch von Leberzellen, den Hepatozyten, aufgenommen wird, sich im Funktionsgewebe (Parenchym) anreichert und den Kontrast in gesundem Lebergewebe verstärkt. Es ist für die Verwendung in der Magnetresonanztomographie zugelassen. Für die Computertomographie ist kein vergleichbares hepatobiliäres Kontrastmittel bekannt. Die gilt auch für Gadofosveset, ein intravaskuläres MR-Kontrastmittel auf Gadoliniumbasis, das an Serumalbumin im Blut bindet und damit zu einer langen Verweildauer des Kontrastmittels im Blutkreislauf führt (Halbwertszeit im Blut etwa 17 Stunden). Ein weiteres Beispiel eines intravaskulären Kontrastmittels, das in der Magnetresonanztomographie Verwendung findet, für das es aber in der Computertomographie kein vergleichbares Äquivalent gibt, ist Ferumoxytol, ein kolloidaler Eisen-Kohlenhydrat-Komplex. Ferumoxytol kann als intravenöse Injektion verabreicht werden und ist als Lösung zur intravenösen Injektion unter dem Markennamen Rienso^{®} oder Ferahme^{®} im Handel erhältlich. Der Eisen-Kohlenhydrat-Komplex weist superparamagnetische Eigenschaften auf und kann daher (off-label) zur Kontrastverstärkung bei MR-Untersuchungen eingesetzt werden (siehe z.B.: L.P. Smits et al.: Evaluation of ultrasmall superparamagnetic iron-oxide (USPIO) enhanced MRI with ferumoxytol to quantify arterial wall inflammation, Atherosclerosis 2017, 263: 211-218).

Es kann ein Modell des maschinellen Lernens trainiert werden, den Effekt zunehmender Mengen eines MR-Kontrastmittels auf eine Repräsentation eines Untersuchungsbereichs in einer computertomograpischen Untersuchung zu lernen.

Dem Modell des maschinellen Lernens können Referenz-Repräsentationen des Untersuchungsbereichs einer Vielzahl von Untersuchungsobjekten präsentiert werden, die den Untersuchungsbereich in einer CT-Untersuchung nach der Applikation unterschiedlicher Mengen eines MR-Kontrastmittels repräsentieren. Dabei kann die maximale Menge, die appliziert worden ist, beispielsweise die Standarmenge des MR-Kontrastmittels sein, wie sie für seine Verwendung in der MRT empfohlen oder zugelassen ist.

Ist das Modell des maschinellen Lernens trainiert, können Repräsentationen vorhergesagt werden, die den Untersuchungsbereich in einer CT-Untersuchung nach der Applikation einer größeren Menge des MR-Kontrastmittels als der Standardmenge repräsentieren.

Die vorliegende Erfindung kann ganz oder teilweise mit Hilfe eines Computersystems ausgeführt werden.

Fig. 6 zeigt beispielhaft und schematisch ein solches erfindungsgemäßes Computersystem.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 6 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert
- eine Repräsentation eines Untersuchungsbereichs zu empfangen,
   ∘ wobei die Repräsentation den Untersuchungsbereich ohne Kontrastmittel oder nach einer Applikation einer ersten Menge eines Kontrastmittels repräsentiert,
   ∘ wobei der Untersuchungsbereich die Leber oder ein Teil der Leber eines Menschen ist, wobei die Repräsentation R*ₚ* eine radiologische Aufnahme ist,
- die Repräsentation einem trainierten Modell des maschinellen Lernens zuzuführen,
   ∘ wobei das Modell des maschinellen Lernens auf Basis von Trainingsdaten trainiert worden ist, ausgehend von einer ersten Referenz-Repräsentation R₁ eine Zahl *n-*1 von vorhergesagten Referenz-Repräsentationen R₂^{*} bis R*ₙ*^{*} zu erzeugen, wobei *n* eine ganze Zahl größer als 2 ist,
   ∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
   ∘ wobei die Mengen *a*₁ bis *aₙ* eine Folge von vorzugsweise zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
   ∘ wobei das Trainieren des Modells des maschinellen Lernens ferner die Schritte umfasst:
      - für jede vorhergesagte Referenzrepräsentation Rⱼ^{*}: Berechnen eines Fehlerwerts für jedes Paar aus einer Referenz-Repräsentation Rⱼ und der vorhergesagten Referenz-Repräsentationen Rⱼ^{*} mit Hilfe einer Fehlerfunktion, wobei j ein Index ist, der die Zahlen von 2 bis n durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,
- von dem Modell des maschinellen Lernens eine oder mehrere vorhergesagte Repräsentationen des Untersuchungsbereichs zu empfangen, wobei jede der einen oder der mehreren vorhergesagten Repräsentationen den Untersuchungsbereich nach der Applikation einer unterschiedlichen Menge des Kontrastmittels repräsentiert,
- die eine oder die mehreren vorhergesagten Repräsentationen zu speichern und/oder auszugeben und/oder an ein separates Computersystem zu übermitteln.

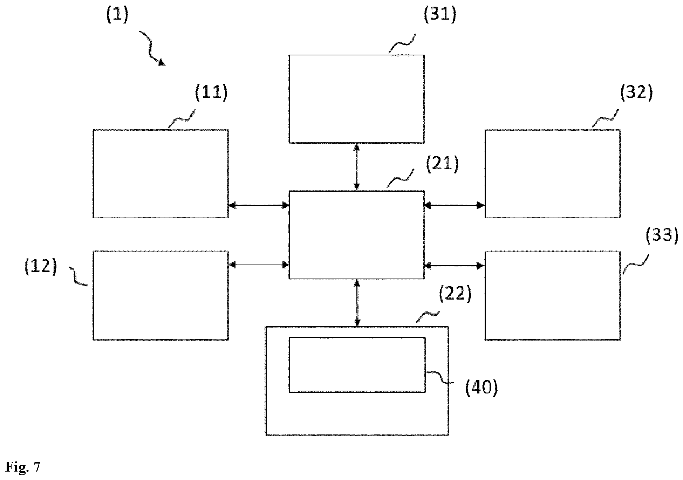

Fig. 7 zeigt beispielhaft und schematisch eine weitere Ausführungsform des erfindungsgemäßen Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Berechnungseinheit, wie sie in Fig. 7 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das erfindungsgemäße System kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

## Patentansprüche

1. Computer-implementiertes Verfahren umfassend die Schritte:
- Empfangen von Trainingsdaten,
∘ wobei die Trainingsdaten eine Vielzahl von Datensätzen einer Vielzahl von Referenz-Untersuchungsobjekten umfassen,
∘ wobei die Datensätze Referenz-Repräsentationen eines Untersuchungsbereichs umfassen, wobei der Untersuchungsbereich die Leber oder ein Teil der Leber eines Menschen ist, wobei die Referenz-Repräsentationen radiologische Aufnahmen sind,
∘ wobei jeder Datensatz eine oder mehrere Referenz-Repräsentationen einer Zahl *n* an Referenz-Repräsentationen umfasst, wobei *n* eine ganze Zahl größer als 2 ist,
∘ wobei jede Referenz-Repräsentation R*ᵢ* den Untersuchungsbereich nach Applikation einer Menge *a*ᵢ eines Kontrastmittels repräsentiert, wobei *i* ein Index ist, der ganze Zahlen von 1 bis *n* durchläuft, wobei die Mengen *a*₁ bis *aₙ* eine Folge von zunehmenden Mengen bilden,
- Trainieren des Modells des maschinellen Lernens,
∘ wobei das Modell trainiert wird, ausgehend von der Referenz-Repräsentation R₁ nacheinander vorhergesagte Referenz-Repräsentationen R₂^{*} bis R*ₙ*^{*} zu erzeugen,
∘ wobei die Erzeugung der vorhergesagten Referenz-Repräsentation R₂^{*} zumindest anteilig auf Basis der Referenz-Repräsentation R₁ basiert und die Erzeugung jeder nachfolgenden vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis n durchläuft,
∘ wobei die vorhergesagte Referenz-Repräsentation R*ₖ*^{*} den Untersuchungsbereich nach Applikation der Menge *a*₁ des Kontrastmittels repräsentiert und die vorhergesagte Referenz-Repräsentation R_{*k*-1}* den Untersuchungsbereich nach Applikation der Menge *a*_{*k*-1} des Kontrastmittels repräsentiert,
∘ wobei das Trainieren des Modells des maschinellen Lernens ferner die Schritte umfasst:
• für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei j ein Index ist, der die Zahlen von 2 bis n durchläuft,
• Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
• Modifizieren von Parametern des Modells des maschinellen Lernens, so dass der Gesamtfehlerwert auf ein definiertes Minimum reduziert wird,
- Nutzen des trainierten Modells des maschinellen Lernens zur Vorhersage.

2. Verfahren gemäß Anspruch 1, ferner umfassend die Schritte:
• Empfangen einer Repräsentation R*ₚ* des Untersuchungsbereichs, wobei die Repräsentation R*ₚ* den Untersuchungsbereich ohne Kontrastmittel oder nach Applikation der Menge *aₚ* des Kontrastmittels repräsentiert, wobei *p* eine ganze Zahl ist, die kleiner als *n* ist,
• Zuführen der Repräsentation R*ₚ* dem trainierten Modell des maschinellen Lernens,
• Empfangen, von dem Modell des maschinellen Lernens, einer oder mehrerer vorhergesagter Repräsentationen *R_{p+q}*^{*} des Untersuchungsbereichs, wobei jede der einen oder der mehreren vorhergesagten Repräsentationen *R_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
• Ausgeben und/oder Speichern und/oder Übermitteln der einen oder der mehreren vorhergesagten Repräsentationen *R_{p+q}*^{*}.

3. Verfahren gemäß Anspruch 2, wobei jede von dem Modell des maschinellen Lernens erzeugte vorhergesagte Repräsentation *R_{p+q}*^{*} wieder dem trainierten Modell des maschinellen Lernens zugeführt wird, um eine vorhergesagte Repräsentation R_{*p+q+*1}^{*} zu erzeugen.

4. Verfahren gemäß Anspruch 3, wobei *m* im Bereich von *n* bis *n+10* liegt.

5. Verfahren gemäß einem der Ansprüche 1 bis 4, wobei es sich bei den Referenz-Repräsentationen und den vorhergesagten Referenz-Repräsentationen und der Repräsentation R*ₚ* und den vorhergesagten Repräsentationen *R_{p+q}*^{*} und R_{*p+q+*1}^{*} um CT-Aufnahmen, MRT-Aufnahmen oder Ultraschallaufnahmen handelt.

6. Verfahren gemäß einem der Ansprüche 1 bis 4, wobei es sich bei den Referenz-Repräsentationen und den vorhergesagten Referenz-Repräsentationen und der Repräsentation R*ₚ* und den vorhergesagten Repräsentationen *R_{p+q}*^{*} und R_{*p+q+*1}^{*} um CT-Aufnahmen handelt und wobei es sich bei dem Kontrastmittel um ein MR-Kontrastmittel handelt.

7. Verfahren gemäß Anspruch 6, wobei die Gesamtfehlerfunktion folgende Formel hat: $LV = {\sum }_{j = 2}^{n} {w}_{j} ⋅ {LF}_{j}$ wobei LV der Gesamtfehlerwert ist, wobei LF*ⱼ* die Fehlerfunktionen für die Berechnung der Fehlerwerte für die Abweichungen zwischen der Referenz-Repräsentation R*ⱼ* und der vorhergesagten Referenz-Repräsentation R*ⱼ*^{*} ist, und wⱼ die Gewichtsfaktoren sind, wobei *j* ein Index ist, der die Zahlen von 2 bis *n* durchläuft.

8. Verfahren gemäß einem der Ansprüche 1 bis 7, wobei jede vorhergesagte Repräsentation und Referenz-Repräsentation nach der folgenden Formel berechnet wird: ${M}^{q} \left({R}_{1}\right) = {R}_{1 + q} *$ wobei M das Modell des maschinellen Lernens repräsentiert, wobei **M***^{q}* die q-fache Anwendung des Modells des maschinellen Lernens bedeutet, und q eine ganze Zahl ist, die die Werte 1 bis *m* annehmen kann, wobei *m* eine ganze Zahl ist, die kleiner als *n-*1 ist oder gleich *n-*1 ist oder größer als *n-*1 ist.

9. Computersystem umfassend
• eine Eingabeeinheit,
• eine Steuer- und Recheneinheit und
• eine Ausgabeeinheit,
wobei die Steuer- und Recheneinheit konfiguriert ist,
• eine Repräsentation R*ₚ* eines Untersuchungsbereichs zu empfangen,
∘ wobei die Repräsentation R*ₚ* den Untersuchungsbereich ohne Kontrastmittel oder nach einer Applikation einer Menge *aₚ* eines Kontrastmittels repräsentiert, wobei *p* eine ganze Zahl ist, die kleiner als *n* ist, wobei *n* eine ganze Zahl größer als 2 ist,
∘ wobei der Untersuchungsbereich die Leber oder ein Teil der Leber eines Menschen ist, wobei die Repräsentation R*ₚ* eine radiologische Aufnahme ist,
• die Repräsentation R*ₚ* einem trainierten Modell des maschinellen Lernens zuzuführen,
∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft, wobei die Mengen *a*₁ bis *aₙ* eine Folge von zunehmenden Mengen bilden,
∘ wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
∘ wobei das Trainieren des Modells des maschinellen Lernens ferner die Schritte umfasst:
- für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,
• von dem Modell des maschinellen Lernens eine oder mehrere vorhergesagte Repräsentationen R*_{p+q}*^{*} des Untersuchungsbereichs zu empfangen, wobei jede der einen oder der mehreren vorhergesagten Repräsentationen R*_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
• die eine oder die mehreren vorhergesagten Repräsentationen R*_{p+q}*^{*} zu speichern und/oder auszugeben und/oder an ein separates Computersystem zu übermitteln.

10. Computerprogrammprodukt umfassend ein Computerprogramm, das in einen Arbeitsspeicher eines Computersystems geladen werden kann und dort das Computersystem dazu veranlasst, folgende Schritte ausführen:
• Empfangen einer Repräsentation R*ₚ* eines Untersuchungsbereichs,
∘ wobei die Repräsentation R*ₚ* den Untersuchungsbereich ohne Kontrastmittel oder nach Applikation einer Menge *aₚ* eines Kontrastmittels repräsentiert, wobei *p* eine ganze Zahl ist, die kleiner als *n* ist, wobei *n* eine ganze Zahl größer als 2 ist,
∘ wobei der Untersuchungsbereich die Leber oder ein Teil der Leber eines Menschen ist, wobei die Repräsentation R*ₚ* eine radiologische Aufnahme ist,
• Zuführen der Repräsentation R*ₚ* einem trainierten Modell des maschinellen Lernens,
∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
∘ wobei das Trainieren des Modells des maschinellen Lernens ferner die Schritte umfasst:
- für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,
• Empfangen, von dem Modell des maschinellen Lernens, einer oder mehrerer vorhergesagter Repräsentationen R*_{p+q}*^{*} des Untersuchungsbereichs, wobei jede der einen oder der mehreren vorhergesagten Repräsentationen R*_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
• Ausgeben und/oder Speichern und/oder Übermitteln der einen oder der mehreren vorhergesagten Repräsentationen R*_{p+q}*^{*}.

11. Verwendung eines Kontrastmittels in einem radiologischen Untersuchungsverfahren, wobei das radiologische Untersuchungsverfahren die folgenden Schritte umfasst:
• Empfangen einer Repräsentation R*ₚ* eines Untersuchungsbereichs,
∘ wobei die Repräsentation R*ₚ* den Untersuchungsbereich ohne Kontrastmittel oder nach Applikation einer Menge *aₚ* des Kontrastmittels repräsentiert, wobei *p* eine ganze Zahl ist, die kleiner als *n* ist, wobei *n* eine ganze Zahl größer als 2 ist,
∘ wobei der Untersuchungsbereich die Leber oder ein Teil der Leber eines Menschen ist, wobei die Repräsentation R*ₚ* eine radiologische Aufnahme ist,
• Zuführen der Repräsentation R*ₚ* einem trainierten Modell des maschinellen Lernens,
∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei k ein Index ist, der ganze Zahlen von 3 bis *n* durchläuft,
∘ wobei das Trainieren des Modells des maschinellen Lernens ferner die Schritte umfasst:
- für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,
• Empfangen, von dem Modell des maschinellen Lernens, einer oder mehrerer vorhergesagter Repräsentationen *R_{p+q}*^{*} des Untersuchungsbereichs, wobei jede der einen oder der mehreren vorhergesagten Repräsentationen *R_{p+q}*^{*} den Untersuchungsbereich nach der Applikation der Menge *a_{p+q}* des Kontrastmittels repräsentiert, wobei q ein Index ist, der die Zahlen von 1 bis *m* durchläuft, wobei *m* eine ganze Zahl ist, die kleiner als *n-1* ist oder gleich *n-1* ist oder größer als *n-1* ist,
• Ausgeben und/oder Speichern und/oder Übermitteln der einen oder der mehreren vorhergesagten Repräsentationen *R_{p+q}*^{*}.

12. Kit umfassend ein Kontrastmittel und ein Computerprogrammprodukt, wobei das Computerprogrammprodukt ein Computerprogramm umfasst, das in einen Arbeitsspeicher eines Computersystems geladen werden kann und dort das Computersystem dazu veranlasst, folgende Schritte ausführen:
• Empfangen einer Repräsentation R*ₚ* eines Untersuchungsbereichs,
∘ wobei die Repräsentation R*ₚ* den Untersuchungsbereich ohne Kontrastmittel oder nach Applikation einer Menge *aₚ* des Kontrastmittels repräsentiert, wobei *p* eine ganze Zahl ist, die kleiner als *n* ist, wobei *n* eine ganze Zahl größer als 2 ist,
∘ wobei der Untersuchungsbereich die Leber oder ein Teil der Leber eines Menschen ist, wobei die Repräsentation R*ₚ* eine radiologische Aufnahme ist,
• Zuführen der Repräsentation R*ₚ* einem trainierten Modell des maschinellen Lernens,
∘ wobei das Modell des maschinellen Lernens auf Basis von Trainingsdaten trainiert worden ist, ausgehend von einer ersten Referenz-Repräsentation R₁ eine Zahl *n-1* von vorhergesagten Referenz-Repräsentationen R₂^{*} bis R*ₙ*^{*} zu erzeugen,
∘ wobei die erste Referenzrepräsentation R₁ den Untersuchungsbereich ohne Kontrastmittel oder nach Applikation einer ersten Menge *a*₁ des Kontrastmittels repräsentiert und jede vorhergesagte Referenz-Repräsentation R*ⱼ*^{*} den Untersuchungsbereich nach einer Menge *aⱼ* des Kontrastmittels repräsentiert, wobei *j* ein Index ist, der ganze Zahlen von 2 bis *n* durchläuft,
∘ wobei die Mengen *a*₁ bis *aₙ* eine Folge von zunehmenden Mengen bilden, wobei die Erzeugung der vorhergesagten zweiten Referenz-Repräsentation R₂^{*} zumindest anteilig auf Basis der Referenz-Repräsentation R₁ erfolgt und die Erzeugung jeder weiteren vorhergesagten Referenz-Repräsentation R*ₖ*^{*} zumindest anteilig auf Basis der vorhergesagten Referenz-Repräsentation R_{*k*-1}^{*} erfolgt, wobei *k* ein Index ist, der ganze Zahlen von 3 bis *n* durchläuft,
∘ wobei das Trainieren des Modells des maschinellen Lernens ferner die Schritte umfasst:
- für jede vorhergesagte Referenzrepräsentation R*ⱼ*^{*}: Berechnen eines Fehlerwerts für jedes Paar aus einer Referenz-Repräsentation R*ⱼ* und der vorhergesagten Referenz-Repräsentationen R*ⱼ*^{*} mit Hilfe einer Fehlerfunktion, wobei *j* ein Index ist, der die Zahlen von 2 bis *n* durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,
• Empfangen, von dem Modell des maschinellen Lernens, einer oder mehrerer vorhergesagter Repräsentationen *R_{p+q}*^{*} des Untersuchungsbereichs, wobei jede der einen oder der mehreren vorhergesagten Repräsentationen *R_{p+q}*^{*} den Untersuchungsbereich nach der Applikation der Menge *a_{p+q}* des Kontrastmittels repräsentiert, wobei *q* ein Index ist, der die Zahlen von 1 bis *m* durchläuft, wobei *m* eine ganze Zahl ist, die kleiner als *n-1* ist oder gleich *n-1* ist oder größer als *n-1* ist,
• Ausgeben und/oder Speichern und/oder Übermitteln der einen oder der mehreren vorhergesagten Repräsentationen *R_{p+q}*^{*}.

## Claims

1. A computer-implemented method comprising the steps of:
- receiving training data,
∘ wherein the training data comprise a plurality of data sets of a plurality of reference examination objects,
∘ wherein the data sets comprise reference representations of an examination area, wherein the examination area is the liver or a part of the liver of a human being, wherein the reference representations are radiological images,
∘ wherein each data set comprises one or more reference representations of a number *n* of reference representations, wherein *n* is an integer greater than 2,
∘ wherein each reference representation R*ᵢ* represents the examination area after application of an amount *aᵢ* of a contrast agent, wherein *i* is an index running through integers from 1 to *n*, wherein the amounts *a*₁ to *aₙ* form a sequence of increasing amounts,
- training the machine learning model,
∘ wherein the model is trained to generate, starting from the reference representation R₁, predicted reference representations R₂^{*} to R*ₙ*^{*} in succession,
∘ wherein the generation of the predicted reference representation R₂ is based at least in part on the reference representation R₁, and the generation of each subsequent predicted reference representation R*ₖ* is based at least in part on the predicted reference representation R_{*k*-1}^{*}, wherein *k* is an index running through integers from 3 to *n*,
∘ wherein the predicted reference representation R*ₖ* represents the examination area after application of the amount *aₖ* of the contrast agent and the predicted reference representation R_{*k*-1} represents the examination area after application of the amount *a*_{*k*-1} of the contrast agent,
∘ wherein the training of the machine learning model further comprises the steps of:
• for each predicted reference representation R*ⱼ*: calculating a loss value for each pair of a reference representation R*ⱼ* and the predicted reference representation R*ⱼ* by means of a loss function, wherein *j* is an index running through the numbers from 2 to *n*,
• calculating a total loss value by means of a total loss function, wherein the total loss function is a function of the loss values, in which the loss values are weighted with weighting factors,
• modifying parameters of the machine learning model such that the total loss value is reduced to a defined minimum,
- using the trained machine learning model for prediction.

2. The method according to claim 1, further comprising the steps of:
• receiving a representation R*ₚ* of the examination area, wherein the representation R*ₚ* represents the examination area without contrast agent or after application of the amount *aₚ* of the contrast agent, wherein *p* is an integer that is smaller than *n*,
• feeding the representation R*ₚ* to the trained machine learning model,
• receiving, from the machine learning model, one or more predicted representations R_{*p*+*q*} of the examination area, wherein each of the one or more predicted representations R_{*p*+*q*} represents the examination area after the application of the amount *a*_{*p*+*q*} of the contrast agent, wherein *q* is an index running through the numbers from 1 to *m*, wherein *m* is an integer that is smaller than *n*-1 or equal to *n*-1 or greater than *n*-1,
• outputting and/or storing and/or transmitting the one or more predicted representations R_{*p*+*q*}^{*}.

3. The method according to claim 2, wherein each predicted representation R_{*p*+q} generated by the machine learning model is fed back to the trained machine learning model to generate a predicted representation R_{*p*+*q*+1}.

4. The method according to claim 3, wherein *m* is in the range from *n* to *n*+10.

5. The method according to any one of claims 1 to 4, wherein the reference representations and the predicted reference representations and the representation R*ₚ* and the predicted representations R_{*p*+*q*} and R_{*p*+*q*+1} are CT images, MRI images or ultrasound images.

6. The method according to any one of claims 1 to 4, wherein the reference representations and the predicted reference representations and the representation R*ₚ* and the predicted representations R_{*p*+*q*} and R_{*p*+*q*+1} are CT images and wherein the contrast agent is an MR contrast agent.

7. The method according to claim 6, wherein the total loss function has the following formula: $LV = {\sum }_{j = 2}^{n} {w}_{j} ⋅ {LF}_{j}$ wherein LV is the total loss value, wherein LF*ⱼ* are the loss functions for calculating the loss values for the deviations between the reference representation R*ⱼ* and the predicted reference representation R*ⱼ*^{*}, and w*ⱼ* are the weighting factors, wherein *j* is an index running through the numbers from 2 to *n*.

8. The method according to any one of claims 1 to 7, wherein each predicted representation and each predicted reference representation is calculated according to the following formula: ${M}^{q} \left({R}_{1}\right) = {R}_{1 + q} *$ wherein **M** represents the machine learning model, wherein **M***^{q}* denotes the *q*-fold application of the machine learning model, and *q* is an integer that can assume values from 1 to *m*, wherein *m* is an integer that is smaller than *n*-1 or equal to *n*-1 or greater than *n*-1.

9. A computer system comprising
• an input unit,
• a control and processing unit, and
• an output unit,
wherein the control and processing unit is configured to:
• receive a representation R*ₚ* of an examination area,
∘ wherein the representation R*ₚ* represents the examination area without contrast agent or after an application of an amount *aₚ* of a contrast agent, wherein *p* is an integer that is smaller than *n,* wherein *n* is an integer greater than 2,
∘ wherein the examination area is the liver or a part of the liver of a human being, wherein the representation R*ₚ* is a radiological image,
• feed the representation R*ₚ* to a trained machine learning model,
∘ wherein the machine learning model has been trained on the basis of training data to generate, starting from a first reference representation R₁, a number *n-1* of predicted reference representations R₂^{*} to R*ₙ*^{*},
∘ wherein the first reference representation R₁ represents the examination area without contrast agent or after application of a first amount *a*₁ of the contrast agent and each predicted reference representation R*ⱼ*^{*} represents the examination area after an amount *aⱼ* of the contrast agent, wherein *j* is an index running through integers from 2 to *n,* wherein the amounts *a*₁ to *aₙ* form a sequence of increasing amounts,
o wherein the generation of the predicted second reference representation R₂ is based at least in part on the reference representation R₁ and the generation of each further predicted reference representation R*ₖ* is based at least in part on the predicted reference representation R_{*k*-1}^{*}, wherein *k* is an index running through integers from 3 to *n,*
o wherein the training of the machine learning model further comprises the steps of:
- for each predicted reference representation R*ⱼ*: calculating a loss value for each pair of a reference representation R*ⱼ* and the predicted reference representation R*ⱼ* by means of a loss function, wherein *j* is an index running through the numbers from 2 to *n,*
- calculating a total loss value by means of a total loss function, wherein the total loss function is a function of the loss values, in which the loss values are weighted with weighting factors,
- modifying parameters of the machine learning model such that the total loss value is reduced to a defined minimum,
• receive, from the machine learning model, one or more predicted representations *R_{p+q}* of the examination area, wherein each of the one or more predicted representations *R_{p+q}* represents the examination area after the application of the amount *a_{p+q}* of the contrast agent, wherein *q* is an index running through the numbers from 1 to *m,* wherein *m* is an integer that is smaller than *n-*1 or equal to *n-*1 or greater than *n*-1,
• store and/or output and/or transmit to a separate computer system the one or more predicted representations *R_{p+q}*^{*}.

10. A computer program product comprising a computer program that can be loaded into a working memory of a computer system and there causes the computer system to carry out the following steps:
• receiving a representation R*ₚ* of an examination area,
∘ wherein the representation R*ₚ* represents the examination area without contrast agent or after application of an amount *aₚ* of a contrast agent, wherein *p* is an integer that is smaller than *n*, wherein *n* is an integer greater than 2,
∘ wherein the examination area is the liver or a part of the liver of a human being, wherein the representation R*ₚ* is a radiological image,
• feeding the representation R*ₚ* to a trained machine learning model,
∘ wherein the machine learning model has been trained on the basis of training data to generate, starting from a first reference representation R₁, a number *n*-1 of predicted reference representations R₂^{*} to R*ₙ*^{*},
∘ wherein the first reference representation R₁ represents the examination area without contrast agent or after application of a first amount *a*₁ of the contrast agent and each predicted reference representation R*ⱼ*^{*} represents the examination area after an amount *aⱼ* of the contrast agent, wherein *j* is an index running through integers from 2 to *n*,
∘ wherein the amounts *a*₁ to *aₙ* form a sequence of increasing amounts, wherein the generation of the predicted second reference representation R₂ is based at least in part on the reference representation R₁ and the generation of each further predicted reference representation R*ₖ* is based at least in part on the predicted reference representation R_{*k*-1}^{*}, wherein *k* is an index running through integers from 3 to *n*,
∘ wherein the training of the machine learning model further comprises the steps of:
- for each predicted reference representation R*ⱼ*: calculating a loss value for each pair of a reference representation R*ⱼ* and the predicted reference representation R*ⱼ* by means of a loss function, wherein *j* is an index running through the numbers from 2 to *n*,
- calculating a total loss value by means of a total loss function, wherein the total loss function is a function of the loss values, in which the loss values are weighted with weighting factors,
- modifying parameters of the machine learning model such that the total loss value is reduced to a defined minimum,
• receiving, from the machine learning model, one or more predicted representations R_{*p*+*q*} of the examination area, wherein each of the one or more predicted representations R_{*p*+*q*} represents the examination area after the application of the amount *a*_{*p*+*q*} of the contrast agent, wherein *q* is an index running through the numbers from 1 to *m*, wherein *m* is an integer that is smaller than *n*-1 or equal to *n*-1 or greater than *n*-1,
• outputting and/or storing and/or transmitting the one or more predicted representations R_{*p*+*q*}^{*}.

11. Use of a contrast agent in a radiological examination method, wherein the radiological examination method comprises the following steps:
• receiving a representation R*ₚ* of an examination area,
∘ wherein the representation R*ₚ* represents the examination area without contrast agent or after application of an amount *aₚ* of the contrast agent, wherein *p* is an integer that is smaller than *n*, wherein *n* is an integer greater than 2,
∘ wherein the examination area is the liver or a part of the liver of a human being, wherein the representation R*ₚ* is a radiological image,
• feeding the representation R*ₚ* to a trained machine learning model,
∘ wherein the machine learning model has been trained on the basis of training data to generate, starting from a first reference representation R₁, a number *n*-1 of predicted reference representations R₂^{*} to R*ₙ*^{*},
∘ wherein the first reference representation R₁ represents the examination area without contrast agent or after application of a first amount *a*₁ of the contrast agent and each predicted reference representation R*ⱼ*^{*} represents the examination area after an amount *aⱼ* of the contrast agent, wherein *j* is an index running through integers from 2 to *n*,
∘ wherein the amounts *a*₁ to *aₙ* form a sequence of increasing amounts, wherein the generation of the predicted second reference representation R₂ is based at least in part on the reference representation R₁ and the generation of each further predicted reference representation R*ₖ* is based at least in part on the predicted reference representation R_{*k*-1}^{*}, wherein *k* is an index running through integers from 3 to *n*,
∘ wherein the training of the machine learning model further comprises the steps of:
- for each predicted reference representation R*ⱼ*: calculating a loss value for each pair of a reference representation R*ⱼ* and the predicted reference representation R*ⱼ* by means of a loss function, wherein *j* is an index running through the numbers from 2 to *n*,
- calculating a total loss value by means of a total loss function, wherein the total loss function is a function of the loss values, in which the loss values are weighted with weighting factors,
- modifying parameters of the machine learning model such that the total loss value is reduced to a defined minimum,
• receiving, from the machine learning model, one or more predicted representations R_{*p*+*q*} of the examination area, wherein each of the one or more predicted representations R_{*p*+*q*} represents the examination area after the application of the amount *a*_{*p*+*q*} of the contrast agent, wherein *q* is an index running through the numbers from 1 to *m*, wherein *m* is an integer that is smaller than *n*-1 or equal to *n*-1 or greater than *n*-1,
• outputting and/or storing and/or transmitting the one or more predicted representations R_{*p*+*q*}^{*}.

12. A kit comprising a contrast agent and a computer program product, wherein the computer program product comprises a computer program that can be loaded into a working memory of a computer system and there causes the computer system to carry out the following steps:
• receiving a representation R*ₚ* of an examination area,
∘ wherein the representation R*ₚ* represents the examination area without contrast agent or after application of an amount *aₚ* of the contrast agent, wherein *p* is an integer that is smaller than *n*, wherein *n* is an integer greater than 2,
∘ wherein the examination area is the liver or a part of the liver of a human being, wherein the representation R*ₚ* is a radiological image,
• feeding the representation R*ₚ* to a trained machine learning model,
∘ wherein the machine learning model has been trained on the basis of training data to generate, starting from a first reference representation R₁, a number *n*-1 of predicted reference representations R₂^{*} to R*ₙ*^{*},
∘ wherein the first reference representation R₁ represents the examination area without contrast agent or after application of a first amount *a*₁ of the contrast agent and each predicted reference representation R*ⱼ*^{*} represents the examination area after an amount *aⱼ* of the contrast agent, wherein *j* is an index running through integers from 2 to *n*,
∘ wherein the amounts *a*₁ to *aₙ* form a sequence of increasing amounts, wherein the generation of the predicted second reference representation R₂ is based at least in part on the reference representation R₁ and the generation of each further predicted reference representation R*ₖ* is based at least in part on the predicted reference representation R_{*k*-1}^{*}, wherein *k* is an index running through integers from 3 to *n*,
∘ wherein the training of the machine learning model further comprises the steps of:
- for each predicted reference representation R*ⱼ*: calculating a loss value for each pair of a reference representation R*ⱼ* and the predicted reference representation R*ⱼ* by means of a loss function, wherein *j* is an index running through the numbers from 2 to *n*,
- calculating a total loss value by means of a total loss function, wherein the total loss function is a function of the loss values, in which the loss values are weighted with weighting factors,
- modifying parameters of the machine learning model such that the total loss value is reduced to a defined minimum,
• receiving, from the machine learning model, one or more predicted representations R_{*p*+*q*} of the examination area, wherein each of the one or more predicted representations R_{*p*+*q*} represents the examination area after the application of the amount *a*_{*p*+*q*} of the contrast agent, wherein *q* is an index running through the numbers from 1 to *m*, wherein *m* is an integer that is smaller than *n*-1 or equal to *n*-1 or greater than *n*-1,
• outputting and/or storing and/or transmitting the one or more predicted representations R_{*p*+*q*}^{*}.

## Revendications

1. Procédé mis en œuvre par ordinateur comprenant les étapes suivantes :
- réception de données d'entraînement,
∘ dans lequel les données d'entraînement comprennent une pluralité de jeux de données d'une pluralité d'objets d'examen de référence,
∘ dans lequel les jeux de données comprennent des représentations de référence d'une zone d'examen, dans lequel la zone d'examen est le foie ou une partie du foie d'un être humain, dans lequel les représentations de référence sont des images radiologiques,
∘ dans lequel chaque jeu de données comprend une ou plusieurs représentations de référence d'un nombre *n* de représentations de référence, dans lequel *n* est un nombre entier supérieur à 2,
∘ dans lequel chaque représentation de référence Rᵢ représente la zone d'examen après application d'une quantité *aᵢ* d'un agent de contraste, dans lequel *i* est un indice parcourant les nombres entiers de 1 à *n*, dans lequel les quantités *a*₁ à *aₙ* forment une séquence de quantités croissantes,
- entraînement du modèle d'apprentissage automatique,
∘ dans lequel le modèle est entraîné pour générer, à partir de la représentation de référence R₁, successivement des représentations de référence prédites R₂^{*} à R*ₙ*^{*},
∘ dans lequel la génération de la représentation de référence prédite R₂ est basée au moins en partie sur la représentation de référence R₁, et la génération de chaque représentation de référence prédite ultérieure Rₖ est basée au moins en partie sur la représentation de référence prédite R_{*k*-1}^{*}, dans lequel *k* est un indice parcourant les nombres entiers de 3 à *n*,
∘ dans lequel la représentation de référence prédite Rₖ représente la zone d'examen après application de la quantité *aₖ* de l'agent de contraste et la représentation de référence prédite Rₖ₋₁ représente la zone d'examen après application de la quantité *a*_{*k*-1} de l'agent de contraste,
∘ dans lequel l'entraînement du modèle d'apprentissage automatique comprend en outre les étapes suivantes :
• pour chaque représentation de référence prédite Rⱼ : calcul d'une valeur d'erreur pour chaque paire d'une représentation de référence R*ⱼ* et de la représentation de référence prédite R*ⱼ* au moyen d'une fonction d'erreur, dans lequel *j* est un indice parcourant les nombres de 2 à *n*,
• calcul d'une valeur d'erreur totale au moyen d'une fonction d'erreur totale, dans lequel la fonction d'erreur totale est une fonction des valeurs d'erreur, dans laquelle les valeurs d'erreur sont pondérées par des facteurs de pondération,
• modification de paramètres du modèle d'apprentissage automatique de sorte que la valeur d'erreur totale soit réduite à un minimum défini,
- utilisation du modèle d'apprentissage automatique entraîné pour la prédiction.

2. Procédé selon la revendication 1, comprenant en outre les étapes suivantes :
• réception d'une représentation R*ₚ* de la zone d'examen, dans lequel la représentation R*ₚ* représente la zone d'examen sans agent de contraste ou après application de la quantité *aₚ* de l'agent de contraste, dans lequel *p* est un nombre entier inférieur à *n*,
• fourniture de la représentation R*ₚ* au modèle d'apprentissage automatique entraîné,
• réception, du modèle d'apprentissage automatique, d'une ou de plusieurs représentations prédites R_{*p*+*q*} de la zone d'examen, dans lequel chacune de la ou des plusieurs représentations prédites R_{*p*+*q*} représente la zone d'examen après l'application de la quantité *a*_{*p*+*q*} de l'agent de contraste, dans lequel *q* est un indice parcourant les nombres de 1 à *m*, dans lequel *m* est un nombre entier inférieur à *n*-1 ou égal à *n*-1 ou supérieur à *n*-1,
• sortie et/ou stockage et/ou transmission de la ou des plusieurs représentations prédites R_{*p*+*q*}^{*}.

3. Procédé selon la revendication 2, dans lequel chaque représentation prédite R_{p+q} générée par le modèle d'apprentissage automatique est réintroduite dans le modèle d'apprentissage automatique entraîné pour générer une représentation prédite R_{*p*+*q*+1}.

4. Procédé selon la revendication 3, dans lequel *m* se situe dans la plage de *n* à *n*+10.

5. Procédé selon l'une quelconque des revendications 1 à 4, dans lequel les représentations de référence et les représentations de référence prédites et la représentation R*ₚ* et les représentations prédites R_{*p*+*q*} et R_{*p*+*q*+1} sont des images CT, des images IRM ou des images échographiques.

6. Procédé selon l'une quelconque des revendications 1 à 4, dans lequel les représentations de référence et les représentations de référence prédites et la représentation R*ₚ* et les représentations prédites R_{*p*+*q*} et R_{*p*+*q*+1} sont des images CT et dans lequel l'agent de contraste est un agent de contraste RM.

7. Procédé selon la revendication 6, dans lequel la fonction d'erreur totale a la formule suivante : $LV = {\sum }_{j = 2}^{n} {w}_{j} ⋅ {LF}_{j}$ dans lequel LV est la valeur d'erreur totale, dans lequel LF*ⱼ* sont les fonctions d'erreur pour le calcul des valeurs d'erreur pour les écarts entre la représentation de référence R*ⱼ* et la représentation de référence prédite R*ⱼ*^{*}, et *wⱼ* sont les facteurs de pondération, dans lequel *j* est un indice parcourant les nombres de 2 à *n*.

8. Procédé selon l'une quelconque des revendications 1 à 7, dans lequel chaque représentation prédite et représentation de référence est calculée selon la formule suivante : ${M}^{q} \left({R}_{1}\right) = {R}_{1 + q} *$ dans lequel **M** représente le modèle d'apprentissage automatique, dans lequel **M***^{q}* désigne l'application *q*-fois du modèle d'apprentissage automatique, et q est un nombre entier pouvant prendre les valeurs de 1 à *m*, dans lequel *m* est un nombre entier inférieur à *n*-1 ou égal à *n*-1 ou supérieur à *n*-1.

9. Système informatique comprenant
• une unité d'entrée,
• une unité de commande et de calcul, et
• une unité de sortie,
dans lequel l'unité de commande et de calcul est configurée pour :
• recevoir une représentation R*ₚ* d'une zone d'examen,
o dans lequel la représentation R*ₚ* représente la zone d'examen sans agent de contraste ou après une application d'une quantité aₚ d'un agent de contraste, dans lequel *p* est un nombre entier inférieur à *n*, dans lequel *n* est un nombre entier supérieur à 2,
∘ dans lequel la zone d'examen est le foie ou une partie du foie d'un être humain, dans lequel la représentation R*ₚ* est une image radiologique,
• fournir la représentation R*ₚ* à un modèle d'apprentissage automatique entraîné,
o dans lequel le modèle d'apprentissage automatique a été entraîné sur la base de données d'entraînement pour générer, à partir d'une première représentation de référence R₁, un nombre *n*-1 de représentations de référence prédites R₂* à *Rₙ*,*
o dans lequel la première représentation de référence R₁ représente la zone d'examen sans agent de contraste ou après application d'une première quantité *a*₁ de l'agent de contraste et chaque représentation de référence prédite R*ⱼ** représente la zone d'examen après une quantité *aⱼ* de l'agent de contraste, dans lequel *j* est un indice parcourant les nombres entiers de 2 à *n*, dans lequel les quantités *a*₁ *à aₙ* forment une séquence de quantités croissantes,
∘ dans lequel la génération de la deuxième représentation de référence prédite R₂ est basée au moins en partie sur la représentation de référence R₁ et la génération de chaque représentation de référence prédite ultérieure Rₖ est basée au moins en partie sur la représentation de référence prédite R_{*k*-1}*, dans lequel *k* est un indice parcourant les nombres entiers de 3 à *n,*
∘ dans lequel l'entraînement du modèle d'apprentissage automatique comprend en outre les étapes suivantes :
- pour chaque représentation de référence prédite Rⱼ : calcul d'une valeur d'erreur pour chaque paire d'une représentation de référence R*ⱼ* et de la représentation de référence prédite R*ⱼ* au moyen d'une fonction d'erreur, dans lequel *j* est un indice parcourant les nombres de 2 à *n,*
- calcul d'une valeur d'erreur totale au moyen d'une fonction d'erreur totale, dans lequel la fonction d'erreur totale est une fonction des valeurs d'erreur, dans laquelle les valeurs d'erreur sont pondérées par des facteurs de pondération,
- modification de paramètres du modèle d'apprentissage automatique de sorte que la valeur d'erreur totale soit réduite à un minimum défini,
• recevoir, du modèle d'apprentissage automatique, une ou plusieurs représentations prédites R_{*p*+*q*} de la zone d'examen, dans lequel chacune de la ou des plusieurs représentations prédites R_{*p*+*q*} représente la zone d'examen après l'application de la quantité *a*_{*p*+*q*} de l'agent de contraste, dans lequel *q* est un indice parcourant les nombres de 1 à *m*, dans lequel *m* est un nombre entier inférieur à *n*-1 ou égal à *n*-1 ou supérieur à *n*-1,
• stocker et/ou sortir et/ou transmettre à un système informatique séparé la ou les plusieurs représentations prédites R_{*p*+*q*}^{*}.

10. Produit-programme d'ordinateur comprenant un programme d'ordinateur pouvant être chargé dans une mémoire vive d'un système informatique et y amenant le système informatique à exécuter les étapes suivantes :
• réception d'une représentation R*ₚ* d'une zone d'examen,
∘ dans lequel la représentation R*ₚ* représente la zone d'examen sans agent de contraste ou après application d'une quantité aₚ d'un agent de contraste, dans lequel *p* est un nombre entier inférieur à *n*, dans lequel *n* est un nombre entier supérieur à 2,
∘ dans lequel la zone d'examen est le foie ou une partie du foie d'un être humain, dans lequel la représentation R*ₚ* est une image radiologique,
• fourniture de la représentation R*ₚ* à un modèle d'apprentissage automatique entraîné,
∘ dans lequel le modèle d'apprentissage automatique a été entraîné sur la base de données d'entraînement pour générer, à partir d'une première représentation de référence R₁, un nombre *n*-1 de représentations de référence prédites R₂^{*} à R*ₙ*^{*},
∘ dans lequel la première représentation de référence R₁ représente la zone d'examen sans agent de contraste ou après application d'une première quantité *a*₁ de l'agent de contraste et chaque représentation de référence prédite R*ⱼ*^{*} représente la zone d'examen après une quantité *aⱼ* de l'agent de contraste, dans lequel *j* est un indice parcourant les nombres entiers de 2 à *n*,
∘ dans lequel les quantités *a*₁ à *aₙ* forment une séquence de quantités croissantes, dans lequel la génération de la deuxième représentation de référence prédite R₂ est basée au moins en partie sur la représentation de référence R₁ et la génération de chaque représentation de référence prédite ultérieure Rₖ est basée au moins en partie sur la représentation de référence prédite R_{*k*-1}^{*}, dans lequel *k* est un indice parcourant les nombres entiers de 3 à *n*,
∘ dans lequel l'entraînement du modèle d'apprentissage automatique comprend en outre les étapes suivantes :
- pour chaque représentation de référence prédite Rⱼ : calcul d'une valeur d'erreur pour chaque paire d'une représentation de référence R*ⱼ* et de la représentation de référence prédite R*ⱼ* au moyen d'une fonction d'erreur, dans lequel *j* est un indice parcourant les nombres de 2 à *n*,
- calcul d'une valeur d'erreur totale au moyen d'une fonction d'erreur totale, dans lequel la fonction d'erreur totale est une fonction des valeurs d'erreur, dans laquelle les valeurs d'erreur sont pondérées par des facteurs de pondération,
- modification de paramètres du modèle d'apprentissage automatique de sorte que la valeur d'erreur totale soit réduite à un minimum défini,
• réception, du modèle d'apprentissage automatique, d'une ou de plusieurs représentations prédites R_{*p*+*q*} de la zone d'examen, dans lequel chacune de la ou des plusieurs représentations prédites R_{*p*+*q*} représente la zone d'examen après l'application de la quantité *a*_{*p*+*q*} de l'agent de contraste, dans lequel *q* est un indice parcourant les nombres de 1 à *m*, dans lequel *m* est un nombre entier inférieur à *n*-1 ou égal à *n*-1 ou supérieur à *n*-1,
• sortie et/ou stockage et/ou transmission de la ou des plusieurs représentations prédites R_{*p*+*q*}^{*}.

11. Utilisation d'un agent de contraste dans un procédé d'examen radiologique, dans lequel le procédé d'examen radiologique comprend les étapes suivantes :
• réception d'une représentation R*ₚ* d'une zone d'examen,
∘ dans lequel la représentation R*ₚ* représente la zone d'examen sans agent de contraste ou après application d'une quantité *aₚ* de l'agent de contraste, dans lequel *p* est un nombre entier inférieur à *n*, dans lequel *n* est un nombre entier supérieur à 2,
∘ dans lequel la zone d'examen est le foie ou une partie du foie d'un être humain, dans lequel la représentation R*ₚ* est une image radiologique,
• fourniture de la représentation R*ₚ* à un modèle d'apprentissage automatique entraîné,
∘ dans lequel le modèle d'apprentissage automatique a été entraîné sur la base de données d'entraînement pour générer, à partir d'une première représentation de référence R₁, un nombre *n*-1 de représentations de référence prédites R₂^{*} à R*ₙ*^{*},
∘ dans lequel la première représentation de référence R₁ représente la zone d'examen sans agent de contraste ou après application d'une première quantité *a*₁ de l'agent de contraste et chaque représentation de référence prédite R*ⱼ*^{*} représente la zone d'examen après une quantité *aⱼ* de l'agent de contraste, dans lequel *j* est un indice parcourant les nombres entiers de 2 à *n*,
∘ dans lequel les quantités *a*₁ à *aₙ* forment une séquence de quantités croissantes, dans lequel la génération de la deuxième représentation de référence prédite R₂ est basée au moins en partie sur la représentation de référence R₁ et la génération de chaque représentation de référence prédite ultérieure Rₖ est basée au moins en partie sur la représentation de référence prédite R_{*k*-1}^{*}, dans lequel *k* est un indice parcourant les nombres entiers de 3 à *n*,
∘ dans lequel l'entraînement du modèle d'apprentissage automatique comprend en outre les étapes suivantes :
- pour chaque représentation de référence prédite Rⱼ : calcul d'une valeur d'erreur pour chaque paire d'une représentation de référence R*ⱼ* et de la représentation de référence prédite R*ⱼ* au moyen d'une fonction d'erreur, dans lequel *j* est un indice parcourant les nombres de 2 à *n*,
- calcul d'une valeur d'erreur totale au moyen d'une fonction d'erreur totale, dans lequel la fonction d'erreur totale est une fonction des valeurs d'erreur, dans laquelle les valeurs d'erreur sont pondérées par des facteurs de pondération,
- modification de paramètres du modèle d'apprentissage automatique de sorte que la valeur d'erreur totale soit réduite à un minimum défini,
• réception, du modèle d'apprentissage automatique, d'une ou de plusieurs représentations prédites R_{*p*+*q*} de la zone d'examen, dans lequel chacune de la ou des plusieurs représentations prédites R_{*p*+*q*} représente la zone d'examen après l'application de la quantité *a*_{*p*+*q*} de l'agent de contraste, dans lequel *q* est un indice parcourant les nombres de 1 à *m*, dans lequel *m* est un nombre entier inférieur à *n*-1 ou égal à *n*-1 ou supérieur à *n*-1,
• sortie et/ou stockage et/ou transmission de la ou des plusieurs représentations prédites R_{*p*+*q*}^{*}.

12. Kit comprenant un agent de contraste et un produit-programme d'ordinateur, dans lequel le produit-programme d'ordinateur comprend un programme d'ordinateur pouvant être chargé dans une mémoire vive d'un système informatique et y amenant le système informatique à exécuter les étapes suivantes :
• réception d'une représentation R*ₚ* d'une zone d'examen,
∘ dans lequel la représentation R*ₚ* représente la zone d'examen sans agent de contraste ou après application d'une quantité *aₚ* de l'agent de contraste, dans lequel *p* est un nombre entier inférieur à *n*, dans lequel *n* est un nombre entier supérieur à 2,
∘ dans lequel la zone d'examen est le foie ou une partie du foie d'un être humain, dans lequel la représentation R*ₚ* est une image radiologique,
• fourniture de la représentation R*ₚ* à un modèle d'apprentissage automatique entraîné,
∘ dans lequel le modèle d'apprentissage automatique a été entraîné sur la base de données d'entraînement pour générer, à partir d'une première représentation de référence R₁, un nombre *n*-1 de représentations de référence prédites R₂^{*} à R*ₙ*^{*},
∘ dans lequel la première représentation de référence R₁ représente la zone d'examen sans agent de contraste ou après application d'une première quantité *a*₁ de l'agent de contraste et chaque représentation de référence prédite R*ⱼ*^{*} représente la zone d'examen après une quantité *aⱼ* de l'agent de contraste, dans lequel *j* est un indice parcourant les nombres entiers de 2 à *n*,
∘ dans lequel les quantités *a*₁ à *aₙ* forment une séquence de quantités croissantes, dans lequel la génération de la deuxième représentation de référence prédite R₂ est basée au moins en partie sur la représentation de référence R₁ et la génération de chaque représentation de référence prédite ultérieure Rₖ est basée au moins en partie sur la représentation de référence prédite R_{*k*-1}^{*}, dans lequel *k* est un indice parcourant les nombres entiers de 3 à *n*,
∘ dans lequel l'entraînement du modèle d'apprentissage automatique comprend en outre les étapes suivantes :
- pour chaque représentation de référence prédite Rⱼ : calcul d'une valeur d'erreur pour chaque paire d'une représentation de référence R*ⱼ* et de la représentation de référence prédite R*ⱼ* au moyen d'une fonction d'erreur, dans lequel *j* est un indice parcourant les nombres de 2 à *n*,
- calcul d'une valeur d'erreur totale au moyen d'une fonction d'erreur totale, dans lequel la fonction d'erreur totale est une fonction des valeurs d'erreur, dans laquelle les valeurs d'erreur sont pondérées par des facteurs de pondération,
- modification de paramètres du modèle d'apprentissage automatique de sorte que la valeur d'erreur totale soit réduite à un minimum défini,
• réception, du modèle d'apprentissage automatique, d'une ou de plusieurs représentations prédites R_{*p*+*q*} de la zone d'examen, dans lequel chacune de la ou des plusieurs représentations prédites R_{*p*+*q*} représente la zone d'examen après l'application de la quantité *a*_{*p*+*q*} de l'agent de contraste, dans lequel *q* est un indice parcourant les nombres de 1 à *m*, dans lequel *m* est un nombre entier inférieur à *n*-1 ou égal à *n*-1 ou supérieur à *n*-1,
• sortie et/ou stockage et/ou transmission de la ou des plusieurs représentations prédites R_{*p*+*q*}^{*}.
